# EUROPEAN PATENT APPLICATION

(11) **EP 4 317 437 A1**
(43) Date of publication of application: **07.02.2024**
(21) Application number: 22779984.8
(22) Date of filing: 11.03.2022
(51) Int. Cl.: C12N 15/53, C12N 1/15, C12N 1/19, C12N 1/21, C12N 5/10, C12N 9/02, C12N 15/31, C12N 15/63, C12P 7/02, C12P 7/24, C12P 13/00, C12N 15/10

(54) **RECOMBINANT POLYPEPTIDE HAVING CARBOXYLIC ACID REDUCING ACTIVITY**

(30) Priority: 30.03.2021 JP 2021057308
(71) Applicant: Asahi Kasei Kabushiki Kaisha, Tokyo 1000006 (JP)
(72) Inventor: ISAKA Koji, Tokyo 100-0006 (JP); MIYATAKE Ryo, Tokyo 100-0006 (JP); KATAYAMA Sayaka, Tokyo 100-0006 (JP)
(74) Representative: D Young & Co LLP
(86) International application number: PCT/JP2022/010912
(87) International publication number: WO 2022/209763

(57) **Abstract**

There are provided a recombinant polypeptide having an excellent carboxylic acid reducing activity, and a production method of an aliphatic compound using the recombinant polypeptide.

A recombinant polypeptide has: (a) an amino acid sequence A having a sequence identity of 60% or higher, 65% or higher, 70% or higher, 75% or higher, 80% or higher, 85% or higher, 88% or higher, 90% or higher, 95% or higher, 97% or higher, 98% or higher, or 99% or higher with an amino acid sequence set forth in SEQ ID NO: 1; (b) a substitution of at least one amino acid at a position corresponding to a substrate-binding site of a polypeptide having an amino acid sequence set forth in SEQ ID NO: 1 in the amino acid sequence A; and (c) a carboxylic acid reducing activity.

## Description

### Technical Field

The present invention relates to a recombinant polypeptide having a carboxylic acid reducing activity, and a production method of an aliphatic compound using the polypeptide.

### Background Art

Due to weather disasters or climate change accompanying global warming in recent years, there is an increasing need for sustainable innovation aiming at symbiosis with the global environment and environmental conservation. Among them, a bioprocess, which is a substance production process in which a renewable raw material can be used and a biological reaction is used, has been greatly expected. So far, fermentation production processes for various chemical products have been developed. For example, fermentation production methods using microorganisms have been studied for production of a carboxylic acid compound used as a polymer raw material, for example, succinic acid, adipic acid, and the like (Patent Literatures 1 and 2).

Carboxylic acid compounds are attractive compounds not only as raw materials for polymers but also raw materials that can be converted into aldehydes, alcohols, and amines (Patent Literatures 3 to 7 and Non Patent Literatures 1 and 2). Since biomass-derived raw materials can be developed into various compounds by converting the carboxyl groups of these carboxylic acid compounds into substituents such as aldehydes, alcohols, and amines, such a conversion technique is required, but the carboxyl groups are in a thermodynamically stable state, and chemical reactions require large energy. From the viewpoint of converting a carboxyl group into another substituent in fermentation production, attempts have been made to search for and apply an enzyme having a carboxylic acid reducing ability (hereinafter, also referred to as "carboxylic acid reductase") (Patent Literatures 8 to 10 and Non Patent Literature 3).

Among the carboxylic acid reductases, an enzyme derived from Mycobacterium abscessus exhibits a carboxyl group reducing activity against a relatively wide range of substrates, and thus, the applicability for obtaining a target compound has been reported (Patent Literature 11 and Non Patent Literatures 4 and 5). However, the carboxylic acid reductase, including the present enzyme, generally has a low reaction rate and is not sufficient for industrial use. Improvement of catalytic activity and improvement of thermal stability of an enzyme have been studied by enzyme modification, but catalytic activity sufficient for industrial use is still not obtained (Non Patent Literature 6). In particular, a substrate having a relatively short carbon chain length tends to decrease the enzyme activity, and improvement of the activity is required.

### Citation List

### Patent Literature

Patent Literature 1: US Patent No. 5,573,931
Patent Literature 2: WO 2012/177721 A
Patent Literature 3: JP 2012-525856 A
Patent Literature 4: JP 2016-538870 A
Patent Literature 5: JP 2016-501031 A
Patent Literature 6: JP 2017-533734 A
Patent Literature 7: JP 2016-533162 A
Patent Literature 8: JP 2011-254747 A
Patent Literature 9: WO 2014/095986 A
Patent Literature 10: JP 2015-512645 A
Patent Literature 11: US 2020/0080064 A

### Non Patent Literature

Non Patent Literature 1: Yu, et. al. Direct biosynthesis of adipic acid from a synthetic pathway in recombinant Escherichia coli, Biotechinology and Bioengineering, Wiley Periodicals, Inc., 2014, vol. 111, pp.2580.
Non Patent Literature 2: Yu Zhou, et. al. Biosynthesis of adipic acid by a highly efficient induction-free system in Escherichia Coli, Journal of Biotechnology, Elsevier, 2020, 8, pp.314-315
Non Patent Literature 3: Napora-Wijata et al. Biocatalytic reduction of Carboxylic acid, Biothechnology Journal, Biotechvisions, 2014,9, pp.822-843
Non Patent Literature 4: Khusnutdinova et al. Exploring Bacterial Carboxylate Reductase for the Reduction of Bifunctional Carboxylic acids, Biothechnology Journal, Wiley-VCH, 2017, 12, 1600751
Non Patent Literature 5: Fedrochuk et al. Site-directed mutagenesis and stability of the carboxylic acid reductase MAB4714 from Mycobacterium abscessus, Journal of Biotechnology, Elsevier, 2019, 303, pp.72-29
Non Patent Literature 6: Fedrchuk et.al. One-pot Biocatalytic Transformation of Adipic Acid to 6-aminocaproic Acid and 1,6-hexamethylenediamine Using Carboxylic Acid Reductases and Transaminases. J. Am. Chem. Soc., 2020, 142, 2, pp.1038-1048

### Summary of Invention

### Technical Problem

An object of the present invention is to provide a recombinant polypeptide having a carboxylic acid reducing activity. In addition, another object of the present invention is to provide a production method of an aliphatic compound using the recombinant polypeptide.

### Solution to Problem

As a result of intensive studies to solve the above problems, the present inventors have found that the enzyme activity can be improved by introducing a mutation into a site estimated to be a substrate-binding site from analysis of a steric structure of an enzyme derived from Mycobacterium abscessus (a polypeptide having an amino acid sequence set forth in SEQ ID NO: 1). That is, the present invention is as follows:
[1] A recombinant polypeptide having:
   (a) an amino acid sequence A having a sequence identity of 60% or higher, 70% or higher, 80% or higher, 85% or higher, 90% or higher, or 95% or higher with an amino acid sequence set forth in SEQ ID NO: 1;
   (b) a substitution of at least one amino acid at a position corresponding to a substrate-binding site of a polypeptide having an amino acid sequence set forth in SEQ ID NO: 1 in the amino acid sequence A; and
   (c) a carboxylic acid reducing activity;
[2] The recombinant polypeptide according to [1], in which the position corresponding to the substrate-binding site of the polypeptide having the amino acid sequence set forth in SEQ ID NO: 1 in (b) is a position corresponding to positions 283, 284, 298, 303, 306, 335, 512, and 926 based on the amino acid sequence set forth in SEQ ID NO: 1;
[3] The recombinant polypeptide according to [1] or [2], in which (b) in the amino acid sequence A, at least two amino acids at the position corresponding to the substrate-binding site of the polypeptide having the amino acid sequence set forth in SEQ ID NO: 1 are substituted;
[4] The recombinant polypeptide according to any one of [1] to [3], in which (b) in the amino acid sequence A, based on the amino acid sequence set forth in SEQ ID NO: 1,
   · positions corresponding to positions 283 and 303 are substituted,
   · positions corresponding to positions 283 and 335 are substituted,
   · positions corresponding to positions 303 and 306 are substituted,
   · positions corresponding to positions 303 and 335 are substituted,
   · positions corresponding to positions 306 and 335 are substituted, or
   · positions corresponding to positions 283 and 303 are substituted;
[5] The recombinant polypeptide according to any one of [1] to [4], in which (b) in the amino acid sequence A, at least one of the following (i) to (v) is satisfied based on the amino acid sequence set forth in SEQ ID NO: 1:
   (i) an amino acid residue at a position corresponding to position 283 is substituted with any of arginine, threonine, and lysine;
   (ii) an amino acid residue at a position corresponding to position 284 is substituted with any of arginine, threonine, and valine;
   (iii) an amino acid residue at a position corresponding to position 303 is substituted with any of cysteine and methionine;
   (iv) an amino acid residue at a position corresponding to position 306 is substituted with any of arginine and lysine; and
   (v) an amino acid residue at a position corresponding to position 335 is substituted with any of arginine, tyrosine, phenylalanine, and methionine;
[6] The recombinant polypeptide according to any one of [1] to [5], in which (b) in the amino acid sequence A, any one of the following (xi) to (xviii) is satisfied based on the amino acid sequence set forth in SEQ ID NO: 1:
   (xi) an amino acid residue at a position corresponding to position 283 is arginine and an amino acid at a position corresponding to position 303 is methionine;
   (xii) an amino acid residue at a position corresponding to position 283 is arginine and an amino acid at a position corresponding to position 303 is cysteine;
   (xiii) an amino acid residue at a position corresponding to position 283 is arginine and an amino acid at a position corresponding to position 335 is phenylalanine;
   (xiv) an amino acid residue at a position corresponding to position 303 is methionine and an amino acid at a position corresponding to position 306 is lysine;
   (xv) an amino acid residue at a position corresponding to position 303 is methionine and an amino acid at a position corresponding to position 335 is phenylalanine;
   (xvi) an amino acid residue at a position corresponding to position 303 is cysteine and an amino acid at a position corresponding to position 306 is lysine;
   (xvii) an amino acid residue at a position corresponding to position 303 is cysteine and an amino acid at a position corresponding to position 335 is phenylalanine; and
   (xviii) an amino acid residue at a position corresponding to position 306 is lysine and an amino acid at a position corresponding to position 335 is phenylalanine;
[7] The recombinant polypeptide according to any one of [1] to [6], in which the carboxylic acid reducing activity of (c) is improved compared to a carboxylic acid reducing activity of the polypeptide having the amino acid sequence set forth in SEQ ID NO: 1;
[8] The recombinant polypeptide according to any one of [1] to [7], in which the recombinant polypeptide is derived from a microorganism of a genus selected from the group consisting of the genus Mycobacterium, the genus Nocardia, the genus Neurospora, and the genus Segniliparus;
[9] The recombinant polypeptide according to any one of [1] to [8], in which the recombinant polypeptide is derived from a microorganism of the genus Mycobacterium;
[10] The recombinant polypeptide according to any one of [1] to [9], in which the carboxylic acid is represented by Formula (I): R¹-(CH₂)ₙ₁-COOH
   (in the formula, R¹ is a methyl group, a carboxyl group, or an aldehyde group, and n¹ is an integer of 1 to 10), or

      Formula (II): R²-(CH₂)ₙ₂-COOH
   (in the formula, R² is hydrogen, a hydroxyl group, or an amino group, and n² is an integer of 2 to 11);
[11] The recombinant polypeptide according to [10], in which in Formula (I), n¹ is an integer of 1 to 7, and
   in Formula (II), n² is an integer of 2 to 8;
[12] The recombinant polypeptide according to [10] or [11], in which in Formula (I), n¹ is an integer of 2 to 4, and
   in Formula (II), n² is an integer of 3 to 5;
[13] A DNA encoding the recombinant polypeptide according to any one of [1] to [12];
[14] A genetically modified plasmid containing the DNA according to [13];
[15] A recombinant microorganism into which the DNA according to [14] is introduced;
[16] The recombinant microorganism according to [15], in which the recombinant microorganism is a microorganism having a production ability of at least one selected from the group consisting of dicarboxylic acids having 4 to 12 carbon atoms;
[17] A culture of the recombinant microorganism according to [15] or [16] and/or an extract of the culture;
[18] A method for producing a target compound, the production method including mixing the recombinant polypeptide according to any one of [1] to [12] or the culture and/or the extract of the culture according to [17] with a substrate compound to obtain a mixed solution;
[19] The method according to [18], in which the substrate compound is represented by

   Formula (I): R¹-(CH₂)ₙ₁-COOH

   (in the formula, R¹ is a methyl group, a carboxyl group, or an aldehyde group, and n¹ is an integer of 1 to 10), or

      Formula (II): R²-(CH₂)ₙ₂-COOH
   (in the formula, R² is hydrogen, a hydroxyl group, or an amino group, and n² is an integer of 2 to 11), and
   the target compound is an aldehyde;
[20] The method according to [19], in which the aldehyde is selected from the group consisting of 4-aminobutan-1-al, 5-aminopentan-1-al, 6-aminohexan-1-al, butanedialdehyde, pentanedialdehyde, hexanedialdehyde, 4-hydroxybutan-1-al, 5-hydroxypentan-1-al, 6-hydroxyhexan-1-al, and glutaraldehyde;
[21] The method according to [18], in which the target compound is an alcohol, and
   the production method includes adding, to the mixed solution, an enzyme having a catalytic activity of converting an aldehyde into an alcohol to obtain a corresponding alcohol; and
[22] The method according to [18], in which the target compound is an amine, and
   the production method includes adding, to the mixed solution, an enzyme having a catalytic activity of converting an aldehyde into an amine to obtain a corresponding amine.

### Advantageous Effects of Invention

According to the present invention, it is possible to provide a recombinant polypeptide having a carboxylic acid reducing activity, and a production method of an aliphatic compound using the recombinant polypeptide.

### Brief Description of Drawings

Fig. 1 is a diagram showing an amino acid sequence (SEQ ID NO: 1) of a wild-type carboxylic acid reductase.
Fig. 2 is a diagram showing a base sequence (SEQ ID NO: 2) of a wild-type carboxylic acid reductase.
Fig. 3 is a diagram showing an amino acid sequence (SEQ ID NO: 3) of a modified carboxylic acid reductase 1.
Fig. 4 is a diagram showing an amino acid sequence (SEQ ID NO: 4) of a modified carboxylic acid reductase 2.
Fig. 5A is a diagram showing an amino acid sequence (SEQ ID NO: 72) of a carboxylic acid reductase derived from Segniliparus rugosus.
Fig. 5B is a diagram showing an alignment result between the amino acid sequence of SEQ ID NO: 1 and the amino acid sequence of the carboxylic acid reductase derived from Segniliparus rugosus having a sequence identity of 61% with SEQ ID NO: 1.
Fig. 5C is a diagram showing a part of an alignment result between the amino acid sequence of SEQ ID NO: 1 and the amino acid sequence of the carboxylic acid reductase derived from Segniliparus rugosus having a sequence similarity of 61% with SEQ ID NO: 1.
Fig. 6 is a schematic diagram of a plasmid of pSK000.
Fig. 7A is a diagram in which enzyme activities (represented by a substrate consumption rate per unit amount of enzyme per unit time (nmol/min/µg enzyme)) of various modified carboxylic acid reductases against a substrate (adipic acid) are compared to an enzyme activity of a wild-type carboxylic acid reductase. In the diagram, "WT" means a wild-type enzyme.
Fig. 7B is a diagram in which enzyme activities (represented by a substrate consumption rate per unit amount of enzyme per unit time (nmol/min/µg enzyme)) of various modified carboxylic acid reductases against a substrate (6-oxohexanoic acid) are compared to an enzyme activity of a wild-type carboxylic acid reductase. In the diagram, "WT" means a wild-type enzyme.
Fig. 7C is a diagram in which enzyme activities (represented by a substrate consumption rate per unit amount of enzyme per unit time (nmol/min/µg enzyme)) of various modified carboxylic acid reductases against a substrate (6-aminocaproic acid) are compared to an enzyme activity of a wild-type carboxylic acid reductase. In the diagram, "WT" means a wild-type enzyme.
Fig. 7D is a diagram in which enzyme activities (represented by a substrate consumption rate per unit amount of enzyme per unit time (nmol/min/µg enzyme)) of various modified carboxylic acid reductases against a substrate (6-hydroxyhexanoic acid) are compared to an enzyme activity of a wild-type carboxylic acid reductase. In the diagram, "WT" means a wild-type enzyme.

### Description of Embodiments

Hereinafter, embodiments for carrying out the present invention will be specifically described. It should be noted that the present invention is not limited to the following embodiments, and various modifications can be made within the scope of the gist of the present invention. In addition, unless otherwise specified, genetic manipulations such as acquisition of DNA and preparation of a vector and transformation described in the present specification can be performed by the methods described in known documents such as Molecular Cloning 4th Edition (Cold Spring Harbor Laboratory Press, 2012), Current Protocols in Molecular Biology (Greene Publishing Associates and Wiley-Interscience), and genetic engineering experimental note (Takaaki Tamura, YODOSHA CO., LTD.). In the present specification, unless otherwise specified, nucleotide sequences are described in the 5' to 3' directions.

A recombinant polypeptide according to the present invention is a polypeptide that has been modified so as to have an improved carboxylic acid reducing activity. In the present specification, regarding the polypeptide, the carboxylic acid reducing activity refers to an activity of converting a carboxyl group of a carboxylic acid into an aldehyde group. Here, the "carboxylic acid" includes a compound containing one or a plurality of carboxyl groups.

The carboxylic acid is represented by, for example, Formula: R-COOH (in the formula, R is a chain or cyclic organic group composed of one or more atoms selected from the group consisting of C, H, O, N, and S).

In one embodiment, the carboxylic acid is represented by:

Formula (I): R¹-(CH₂)ₙ₁-COOH

(in the formula, R¹ is a methyl group, a carboxyl group, or an aldehyde group, and n¹ is an integer of 1 to 10). In Formula (I), n¹ is preferably an integer of 1 to 7, more preferably an integer of 2 to 4, and still more preferably an integer of 3 or 4.

In another embodiment, the carboxylic acid is represented by:

Formula (II): R²-(CH₂)ₙ₂-COOH

(in the formula, R² is hydrogen, a hydroxyl group, or an amino group, and n² is an integer of 2 to 11). In Formula (II), n² is preferably an integer of 2 to 8, more preferably an integer of 3 to 5, and still more preferably an integer of 4 or 5.

The recombinant polypeptide according to the present invention has properties shown in the following (a) to (c):
(a) an amino acid sequence A having a sequence identity of 60% or higher with an amino acid sequence of a wild-type carboxylic acid reductase;
(b) a substitution of at least one amino acid at a position corresponding to a substrate-binding site of a wild-type carboxylic acid reductase in the amino acid sequence A; and
(c) a carboxylic acid reducing activity.

The carboxylic acid reducing activity of (c) is preferably improved compared to a carboxylic acid reducing activity of the polypeptide having the amino acid sequence set forth in SEQ ID NO: 1. Therefore, in a preferred embodiment, the recombinant polypeptide according to the present invention has a carboxylic acid reducing activity improved compared to a carboxylic acid reducing activity of the polypeptide having the amino acid sequence set forth in SEQ ID NO: 1.

The recombinant polypeptide according to the present invention has an amino acid sequence A having a certain percentage or more of a sequence identity with an amino acid sequence of a wild-type carboxylic acid reductase. The wild-type carboxylic acid reductase polypeptide is classified as EC 1.2.1.30.

The wild-type carboxylic acid reductase is not limited, and can be obtained from, for example, a microorganism selected from the group consisting of the genus Mycobacterium, the genus Nocardia, the genus Neurospora, and the genus Segniliparus. The wild-type carboxylic acid reductase can be obtained preferably from a microorganism belonging to the genus Mycobacterium and the genus Nocardia, more preferably from a microorganism belonging to the genus Mycobacterium, and most preferably from Mycobacterium abscessus.

The "wild-type carboxylic acid reductase" is not limited to a polypeptide having the amino acid sequence set forth in SEQ ID NO: 1, and may be an amino acid sequence in which one or a plurality of amino acids are substituted, added, inserted, or deleted in the amino acid sequence set forth in SEQ ID NO: 1. In addition, one or a plurality of amino acids may be added to either or both of the N-terminus and the C-terminus of the polypeptide. Specifically, the "wild-type carboxylic acid reductase" includes a polypeptide having an amino acid sequence set forth in SEQ ID NO: 1, as well as a polypeptide having an amino acid sequence having a sequence identity of 60% or higher with the amino acid sequence set forth in SEQ ID NO: 1.

In the present specification, the "wild-type carboxylic acid reductase" is a polypeptide having an amino acid sequence having a sequence identity of 60% or higher of the amino acid sequence set forth in SEQ ID NO: 1, and is a polypeptide capable of performing a reduction reaction using a carboxylic acid compound as a substrate in the presence of nicotinamide adenine dinucleotide phosphate (NADPH) and adenosine triphosphate (ATP). Therefore, the "carboxylic acid reductase activity" or "carboxylic acid reducing activity" more specifically means an activity capable of performing a reduction reaction using a carboxylic acid compound as a substrate in the presence of NADPH and ATP.

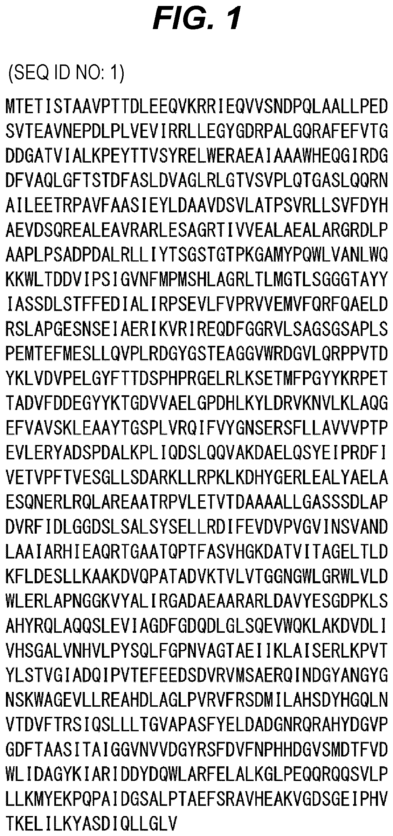

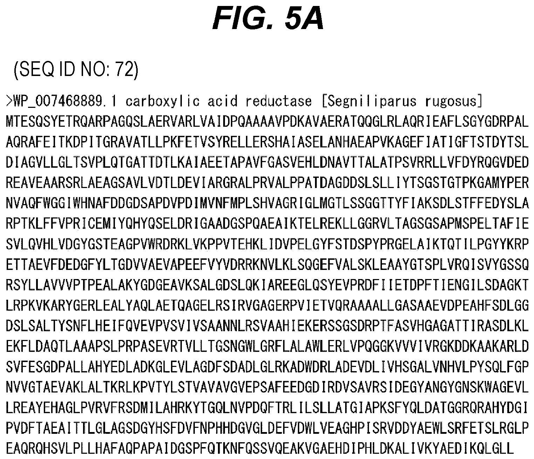

As an example of the wild-type carboxylic acid reductase, an amino acid sequence of a wild-type carboxylic acid reductase derived from Mycobacterium abscessus is set forth in SEQ ID NO: 1 (Fig. 1). As another example of the wild-type carboxylic acid reductase, an amino acid sequence of a wild-type carboxylic acid reductase derived from Segniliparus rugosus is set forth in SEQ ID NO: 72 (Fig. 5A). The enzyme has a sequence identity of 61% with the amino acid sequence set forth in SEQ ID NO: 1.

The recombinant polypeptide according to the present invention has an amino acid sequence A having a certain percentage or more of a sequence identity with an amino acid sequence of a wild-type carboxylic acid reductase. The amino acid sequence A has a sequence identity of 60% or higher, 65% or higher, 70% or higher, 75% or higher, 80% or higher, 85% or higher, 88% or higher, 90% or higher, 95% or higher, 97% or higher, 98% or higher, or 99% or higher with the amino acid sequence set forth in SEQ ID NO: 1.

Regarding the amino acid sequence, the "sequence identity" can be determined by a known method, and is obtained using, for example, an alignment search tool that can be used in Basic Local Alignment Search Tool (BLAST, https://blast.ncbi.nlm.nih.gov/Blast.cgi). The method for determining the sequence identity using this program will be understood by those skilled in the art by reference to the above website, for example, the "help" section. For sequence comparison, Blast2 sequence function of BLAST (registered trademark) (Blastp) program can be used using default parameters (gap existence cost 11 and gap cost 1 per residue). Similarly, a "sequence identity" of a polynucleotide sequence can also be determined by a known method.

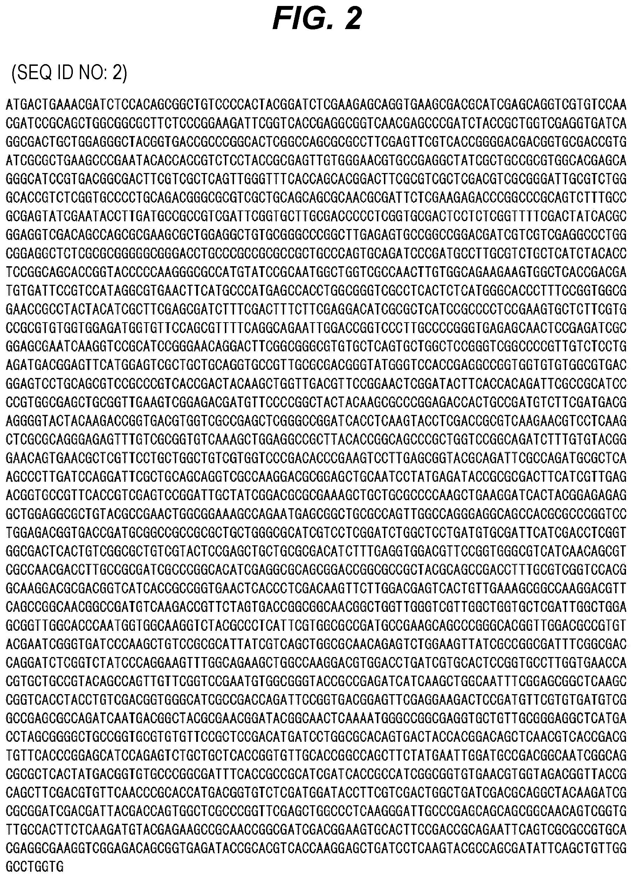

The gene of the wild-type carboxylic acid reductase that can be used in the present invention is, for example, a polynucleotide whose sequence information can be obtained from Genebank gene ID5967171 and which is set forth in, for example, SEQ ID NO: 2 (Fig. 2). The gene can be obtained from Mycobacteroides abscessus ATCC 19977 by a general genetic engineering method. That is, the gene can be obtained as a gene product amplified by PCR using a genomic DNA as a template, and can be used for expression in a host microorganism. In addition, a gene prepared by organic synthesis can also be obtained. In addition, alternative codons that ultimately translate to the same amino acid may also be utilized. Typically, a method for replacing the gene sequence in consideration of the codon usage frequency of the host microorganism is used.

In one embodiment, the recombinant polypeptide according to the present invention has properties shown in the following (a) to (c):
(a) an amino acid sequence A having a sequence identity of 60% or higher with an amino acid sequence set forth in SEQ ID NO: 1;
(b) a substitution of at least one amino acid at a position corresponding to a substrate-binding site of a polypeptide having an amino acid sequence set forth in SEQ ID NO: 1 in the amino acid sequence A; and
(c) a carboxylic acid reducing activity.

Hereinafter, the present invention will be described in detail in the case where the wild-type carboxylic acid reductase is a polypeptide having the amino acid sequence set forth in SEQ ID NO: 1.

At least one amino acid at a position corresponding to a substrate-binding site of a polypeptide having an amino acid sequence set forth in SEQ ID NO: 1 is substituted in the amino acid sequence A. Preferably, at least two amino acids at the positions corresponding to the substrate-binding site of the polypeptide having the amino acid sequence set forth in SEQ ID NO: 1 are substituted in the amino acid sequence A.

Regarding the amino acid sequence A, the "position corresponding to the substrate-binding site of the polypeptide having the amino acid sequence set forth in SEQ ID NO: 1" is a position corresponding to positions 283, 284, 298, 303, 306, 335, 512, and 926 based on the amino acid sequence set forth in SEQ ID NO: 1. Therefore, in the amino acid sequence A, among the amino acids at the positions corresponding to positions 283, 284, 298, 303, 306, 335, 512, and 926 based on the amino acid sequence set forth in SEQ ID NO: 1, one or a plurality of amino acids, preferably at least one amino acid, and more preferably at least two amino acids are substituted. When the amino acid sequence A has the above substitution, an excellent enzyme activity against the target substrate is obtained. Preferably, when the amino acid sequence A has the above substitution, the enzyme activity against the target substrate is improved compared to the wild-type carboxylic acid reductase. A method for specifying the substrate-binding site will be described below.

Regarding the amino acid sequence A, the term "the amino acid residue at the position corresponding to position X (X represents an integer of 1 or more) based on the amino acid sequence set forth in SEQ ID NO: 1" includes both:
· an amino acid residue at a position corresponding to position X based on the amino acid sequence set forth in SEQ ID NO: 1 (case 1); and
· an amino acid residue at position X of the amino acid sequence set forth in SEQ ID NO: 1 (case 2).

Specifically, the term "the amino acid residue at the position corresponding to positions 283, 284, 298, 303, 306, 335, 512, and 926 based on the amino acid sequence set forth in SEQ ID NO: 1" includes both:
· an amino acid residue at a position corresponding to positions 283, 284, 298, 303, 306, 335, 512, and 926 based on the amino acid sequence set forth in SEQ ID NO: 1; and
· an amino acid residue at positions 283, 284, 298, 303, 306, 335, 512, and 926 of the amino acid sequence set forth in SEQ ID NO: 1.

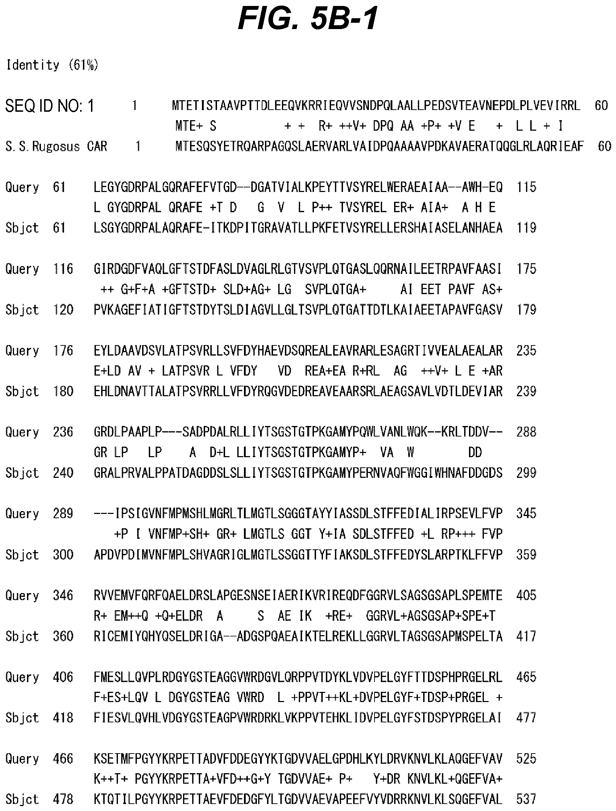

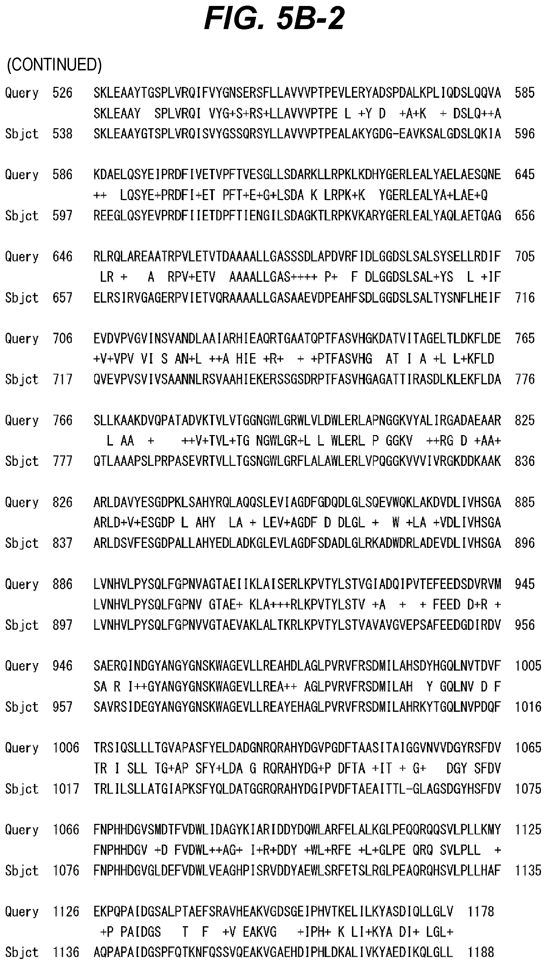

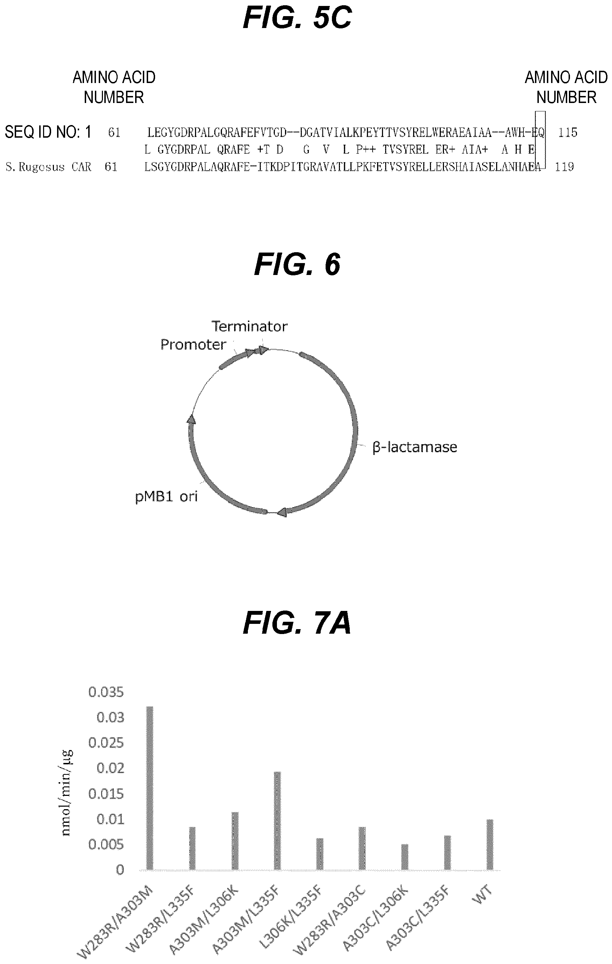

In the present invention, the "amino acid residue at the corresponding position" refers to an amino acid residue in a position corresponding to a specific position in the reference sequence when an amino acid sequence as a reference (specifically, the amino acid sequence set forth in SEQ ID NO: 1, hereinafter, referred to as a reference sequence) and a comparative amino acid sequence (specifically, the amino acid sequence A) are aligned using BLAST (https://blast.ncbi.nlm.nih.gov/Blast.cgi) sequence analysis software. For example, Fig. 5 illustrates the amino acid sequence set forth in SEQ ID NO: 1 and the amino acid sequence of the carboxylic acid derived from Segniliparus rugosus (amino acid sequence of SEQ ID NO: 72, having a sequence identity of 61% with the amino acid sequence set forth in SEQ ID NO: 1) (Fig. 5A), and an alignment result when aligned using BLAST sequence analysis software and a part thereof (amino acid sequence after position 61) (Figs. 5B and 5C). As illustrated in Fig. 5, in the amino acid of a carboxylic acid reductase derived from Segniliparus rugosus, an amino acid residue at a position corresponding to position 115 (glutamine residue in SEQ ID NO: 1) of the amino acid sequence set forth in SEQ ID NO: 1 is an alanine residue at position 119.

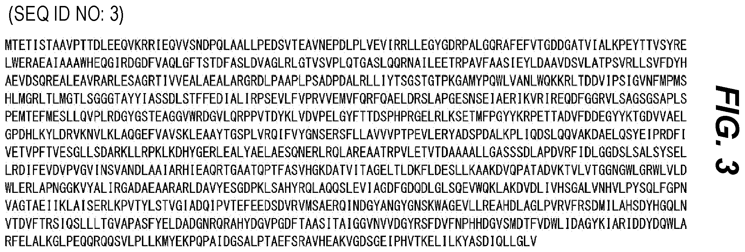

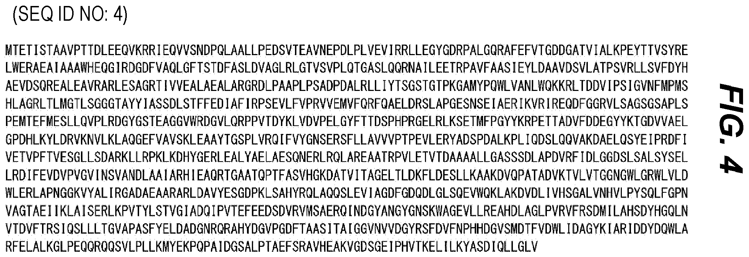

Examples of the recombinant polypeptide (also referred to as "modified carboxylic acid reductase") having the amino acid sequence A according to the present invention are illustrated in Fig. 3 (modified carboxylic acid reductase 1, SEQ ID NO: 3) and Fig. 4 (modified carboxylic acid reductase 2, SEQ ID NO: 4). The modified carboxylic acid reductase 1 (consisting of the polypeptide set forth in SEQ ID NO: 3) illustrated in Fig. 3 has amino acid substitutions of W283R and A303M with respect to SEQ ID NO: 1. The modified carboxylic acid reductase 2 (consisting of the polypeptide set forth in SEQ ID NO: 4) illustrated in Fig. 4 has amino acid substitutions of W283R and L335F with respect to SEQ ID NO: 1. That is, in the modified carboxylic acid reductase 1, the amino acid residue at the position corresponding to position 283 and the amino acid residue at the position corresponding to position 303 are substituted with arginine and methionine, respectively, based on the amino acid sequence set forth in SEQ ID NO: 1. In the modified carboxylic acid reductase 2, the amino acid residue at the position corresponding to position 283 and the amino acid residue at the position corresponding to position 335 are substituted with arginine and phenylalanine, respectively, based on the amino acid sequence set forth in SEQ ID NO: 1.

In one embodiment, in the recombinant polypeptide according to the present invention, the amino acid sequence A satisfies at least one of the following (i) to (v) based on the amino acid sequence set forth in SEQ ID NO: 1:
(i) an amino acid residue at a position corresponding to position 283 is substituted with any of arginine, threonine, and lysine;
(ii) an amino acid residue at a position corresponding to position 284 is substituted with any of arginine, threonine, and valine;
(iii) an amino acid residue at a position corresponding to position 303 is substituted with any of cysteine and methionine;
(iv) an amino acid residue at a position corresponding to position 306 is substituted with any of arginine and lysine; and
(v) an amino acid residue at a position corresponding to position 335 is substituted with any of arginine, tyrosine, phenylalanine, and methionine.

The mutation in the amino acid sequence may be one or a combination of two or more of the substitutions.

Regarding the recombinant polypeptide according to the present invention, in the amino acid sequence A,
· positions corresponding to positions 283 and 303 are substituted based on the amino acid sequence set forth in SEQ ID NO: 1,
· positions corresponding to positions 283 and 335 are substituted based on the amino acid sequence set forth in SEQ ID NO: 1,
· positions corresponding to positions 303 and 306 are substituted based on the amino acid sequence set forth in SEQ ID NO: 1,
· positions corresponding to positions 303 and 335 are substituted based on the amino acid sequence set forth in SEQ ID NO: 1,
· positions corresponding to positions 306 and 335 are substituted based on the amino acid sequence set forth in SEQ ID NO: 1, or
· positions corresponding to positions 283 and 303 are substituted based on the amino acid sequence set forth in SEQ ID NO: 1.

In a preferred embodiment, the recombinant polypeptide according to the present invention has two mutations, and in the amino acid sequence A, at least one of the following (xi) to (xviii) is satisfied based on the amino acid sequence set forth in SEQ ID NO: 1:
(xi) an amino acid residue at a position corresponding to position 283 is arginine and an amino acid at a position corresponding to position 303 is methionine;
(xii) an amino acid residue at a position corresponding to position 283 is arginine and an amino acid at a position corresponding to position 303 is cysteine;
(xiii) an amino acid residue at a position corresponding to position 283 is arginine and an amino acid at a position corresponding to position 335 is phenylalanine;
(xiv) an amino acid residue at a position corresponding to position 303 is methionine and an amino acid at a position corresponding to position 306 is lysine;
(xv) an amino acid residue at a position corresponding to position 303 is methionine and an amino acid at a position corresponding to position 335 is phenylalanine;
(xvi) an amino acid residue at a position corresponding to position 303 is cysteine and an amino acid at a position corresponding to position 306 is lysine;
(xvii) an amino acid residue at a position corresponding to position 303 is cysteine and an amino acid at a position corresponding to position 335 is phenylalanine; and
(xviii) an amino acid residue at a position corresponding to position 306 is lysine and an amino acid at a position corresponding to position 335 is phenylalanine.

The substrate-binding site can be estimated, for example, by existing protein crystal structure information (Protein Data Bank, http://www.rcsb.org/) and enzyme-substrate-binding structure prediction using a computational scientific method.

When enzyme-substrate-binding structure prediction is performed, for example, a crude model based on a crystal structure of an enzyme having a similar amino acid sequence can be acquired using protein three-dimensional structure prediction software represented by swiss-model software (https://swissmodel.expasy.org/). A model of a dynamic stabilization state can be acquired by subjecting the model to molecular dynamics calculation.

It is possible to estimate the substrate-binding site by superimposing the model obtained by the method described above on the predicted existing enzyme-substrate-binding structure. The superposition of protein molecules can be carried out, for example, using the molecular graphic tool Pymol (https://pymol.org/2/) software.

Alternatively, a target substrate can be fitted to the model described above and subjected to molecular dynamics calculation to obtain a dynamic stabilization model of an enzyme-substrate-binding structure. Here, a transition state structure of the substrate in the target reaction is estimated by quantum chemical calculation and is used for enzyme-substrate-binding structure prediction, which is also useful for more accurate binding model prediction. In the resulting enzyme-substrate-binding structure prediction model, an amino acid present in the vicinity of a substrate molecule can be estimated by displaying an amino acid residue present within an arbitrary distance around the substrate molecule using, for example, Pymol software described above.

By preparing and screening a comprehensive mutant enzyme library in which point mutations are introduced into the enzyme amino acids (groups) estimated in this way, it is possible to specify a substrate-binding site that contributes to improvement of enzyme activity.

A second aspect of the present invention is a DNA encoding the recombinant polypeptide. Specifically, it is a DNA encoding the amino acid sequence A. An example of the DNA encoding the recombinant polypeptide is the polynucleotide set forth in SEQ ID NO: 2.

Another aspect of the present invention is a recombinant microorganism into which the DNA encoding the recombinant polypeptide (for example, the polynucleotide set forth in SEQ ID NO: 2) is introduced. Such a recombinant microorganism can be obtained by preparing a recombinant DNA by linking a DNA having a polynucleotide sequence encoding a recombinant polypeptide to a vector DNA, and transforming a strain of a host microorganism using the recombinant DNA.

The host microorganism is, for example, a microorganism instrinsically having an ability to express at least one selected from dicarboxylic acids. That is, the host microorganism intrinsically has a production ability of at least one selected from, for example, dicarboxylic acids. Here, the dicarboxylic acid is preferably a dicarboxylic acid having 4 to 12 carbon atoms, and more preferably a dicarboxylic acid having 4 to 6 carbon atoms. In one embodiment, the host microorganism is more preferably a microorganism intrinsically having an ability to express at least one selected from the group consisting of succinic acid, glutaric acid, and adipic acid.

As the host microorganism, various microorganisms that can be used in a fermentation process are available, and are selected from, for example, bacteria, yeast, and fungi. The host microorganism is selected from, for example, microorganisms of the genera Escherichia, Bacillus, Corynebacterium, Klebsiella, Clostridium, Gluconobacter, Zymomonas, Lactobacillus, Lactococcus, Streptococcus, Pseudomonas, and Streptomyces. The host microorganism is preferably Escherichia Coli from the viewpoint of ease of genetic modification.

The DNA of the recombinant polypeptide according to the present invention may be introduced into a host microorganism using a vector capable of autonomously replicating in the host microorganism, or the DNA of the recombinant polypeptide according to the present invention may be inserted into a chromosome and replicated. The vector is selected, for example, from the group consisting of a plasmid, a phage, a transposon, an IS element, a phasmid, a cosmid, and a linear and circular DNA. The DNA of the recombinant polypeptide is incorporated into a vector and introduced into a host microorganism. The vector is preferably a plasmid or phage.

In a case where the host microorganism is Escherichia coli, a suitable plasmid is, for example, pLG338, pACYC184, pBR322, pUC18, pUC19, pHSG298, pHSG398, pKC30, pRep4, pHS1, pKK223-3, pDHE19.2, pHS2, pPLc236, pMBL24, pLG200, pUR290, pIN-III113-B1, λgt11, or pBdCI. When the host microorganism is a bacillus such as the genus Bacillus, a suitable plasmid is, for example, pUB110, pC194, or pBD214. Other usable plasmids are described in "Gene Cloning and DNA nalysis 6th edition", Wiley-Blackwell, 2016.

Regarding the present invention, a vector can be prepared, for example, by operably linking a promoter (regulatory region) upstream and a terminator downstream of a polynucleotide sequence encoding a gene, respectively, and in some cases, operably linking a gene marker and/or another control sequence.

In the preparation of the recombinant microorganism, an expression mechanism suitable for enhancing the expression of the recombinant polypeptide, for example, a promoter and a terminator can be selected and used. The promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also suitably used in the present invention. In Escherichia coli, a lac system, a trp system, a tac or trc system, or a major operator and promoter region of λ-phage, a regulatory region for fd coat protein, a promoter for a glycolytic enzyme (for example, 3-phosphoglycerate kinase or glyceraldehyde-3-phosphate dehydrogenase), glutamate decarboxylase A, or serine hydroxymethyltransferase, and the like can be used. Examples of the terminator include an rrnBT1T2 terminator and a lac terminator.

Other regulatory elements that can be used in addition to promoters and terminators are, for example, a selectable marker, an amplification signal, and a replication point. A regulatory element is described in, for example, "Gene Expression Technology: Methods in Enzymology 185", Academic Press (1990).

The host microorganism used in the present invention may be a microorganism in which a gene encoding an alcohol dehydrogenase inherent in the microorganism (hereinafter, also referred to as "alcohol dehydrogenase gene") is disrupted. By using such a host microorganism, contamination of an alcohol dehydrogenase unique to the host microorganism can be avoided, and progress of an unintended reaction can be suppressed.

Still another aspect of the present invention relates to the recombinant polypeptide described above or a culture of the recombinant microorganism and/or an extract of the culture.

A desired target compound can be produced using a bacterial cell of a recombinant microorganism expressing the modified carboxylic acid reductase according to the present invention. For example, in a case where the target compound is an alcohol compound, an aldehyde reductase can be added. In a case where the target compound is an amino compound, the amino compound can be produced by adding an aminotransferase or co-expressing an aminotransferase in a host. That is, there is also provided a method for producing a target compound, in which the recombinant microorganism obtained by introducing a DNA encoding a modified carboxylic acid reductase according to the present invention into a host microorganism is cultured, and the culture is used.

Therefore, still another aspect of the present invention relates to a production method of a target compound using the recombinant microorganism described above. Specifically, the production method includes mixing a culture and/or an extract of the culture with a substrate compound to obtain a mixed solution. A reaction time for reacting the culture and/or the extract of the culture with the substrate compound in the mixed solution is a time during which the target product can be produced. The reaction time is, for example, 15 minutes to 48 hours.

Carbon, which is a substrate compound, is represented by, for example,

Formula (I): R¹-(CH₂)ₙ₁-COOH

(in the formula, R¹ is a methyl group, a carboxyl group, or an aldehyde group, and n¹ is an integer of 1 to 10), or

Formula (II): R²-(CH₂)ₙ₂-COOH

(in the formula, R² is hydrogen, a hydroxyl group, or an amino group, and n² is an integer of 2 to 11).

In Formula (I), n¹ is preferably an integer of 1 to 7, and more preferably an integer of 2 to 4. In Formula (II), n² is preferably an integer of 2 to 8, and more preferably an integer of 3 to 5.

In a case where the target compound is an aldehyde, the aldehyde is, for example, selected from the group consisting of 4-aminobutanal, 5-aminopentanal, 6-aminohexanal, butanedialdehyde, pentanedialdehyde, hexanedialdehyde, 4-hydroxybutanal, 5-hydroxypentanal, 6-hydroxyhexanal, 4-oxobutanoic acid, 5-oxopentanoic acid, and 6-oxohexanoic acid. The aldehyde is preferably selected from the group consisting of 6-aminohexanal, hexanedialdehyde, 6-hydroxyhexanal, and 6-oxohexanoic acid.

In another embodiment, the present production method includes adding, to the mixed solution, an enzyme having a catalytic activity of converting an aldehyde into an alcohol to obtain a corresponding alcohol. In the present embodiment, the target compound is an alcohol. The alcohol is, for example, selected from the group consisting of 4-hydroxybutanoic acid, 5-hydroxypentanoic acid, 6-hydroxyhexanoic acid, 4-amino-1-butanol, 5-amino-1-pentanol, 6-amino-1-hexanol, 4-hydroxybutanal, 5-hydroxypentanal, 6-hydroxyhexanal, 1,4-butanediol, 1,5-pentanediol, and 1,6-hexanediol, and is preferably selected from the group consisting of 6-hydroxyhexanoic acid, 6-amino-1-hexanol, 6-hydroxyhexanal, and 1,6-hexanediol.

In still another embodiment, the present production method includes adding, to the mixed solution, an enzyme having a catalytic activity of converting an aldehyde into an amine to obtain a corresponding amine. In the present embodiment, the target compound is an amine. The amine is, for example, selected from the group consisting of 4-aminobutanoic acid, 5-aminopentanoic acid, 6-aminohexanoic acid, 4-aminobutanal, 5-aminopentanal, 6-aminohexanal, 4-amino-1-butanol, 5-amino-1-pentanol, 6-amino-1-hexanol, 1,4-diaminobutane, 1,5-diaminopentane, and 1,6-diaminohexane, and is preferably selected from the group consisting of 6-aminohexanoic acid, 6-aminohexanal, 6-amino-1-hexanol, and 1,6-diaminohexane.

The production method according to the present invention may include culturing the recombinant microorganism described above, accumulating any one of the target compounds in a culture solution, and isolating and purifying the compounds.

The conditions for allowing the carboxylic acid reduction reaction to proceed in the presence of the carboxylic acid reductase according to the present invention may be any conditions under which a target product is produced, and can be set by adjusting the composition, pH, reaction temperature, reaction time, and the like of the reaction solution by a method usually performed by those skilled in the art. Examples of the reaction solution include a buffer solution having a pH of 7 to 9, and more preferably, examples of the reaction solution include a buffer solution containing HEPES-KOH having a pH of 8 to 9.

In the carboxylic acid reductase activity center, an acyl AMP is produced from ATP and a substrate carboxylic acid, and then an acyl group is temporarily translocated to a phosphopantetheinyl group. It is also effective to add a phosphopantetheinyl transferase to the reaction solution for the purpose of supplying a phosphopantetheinyl group to the carboxylic acid reductase. A phosphopantetheinyl transferase polypeptide used in the present invention and a polynucleotide encoding the same are not particularly limited, and polynucleotides obtained from the genus Nocardia (Accession No. DQ904035) and translation products thereof are available. A reaction temperature is usually 20 to 40°C and more preferably 30 to 37°C. A reaction time may be any time as long as the target product can be produced, and is, for example, 15 minutes to 48 hours.

Regarding the culture of the recombinant microorganism and/or the extract of the culture, in the method for preparing an enzyme-containing material containing the recombinant polypeptide according to the present invention, for example, which may be prepared as the enzyme solution, the recombinant microorganism expressing an enzyme according to the present invention can by lysed by, for example, sonication disruption, bead mill disruption, or lysozyme, and the centrifugal supernatant can be used.

A medium composition, culture conditions, and culture time for culturing the recombinant microorganism according to the present invention can be appropriately selected by a method usually performed by those skilled in the art. The culture temperature is typically in a range of 20 to 40°C and preferably 30 to 37°C.

The medium may be a natural, semi-synthetic, or synthetic medium containing one or more carbon sources, nitrogen sources, inorganic salts, vitamins, or optionally trace component such as trace element or vitamin. The medium should adequately meet the nutritional requirements of the transformants to be cultured. Specifically, when the host microorganism is aerobic, shaking should be performed to ensure a suitable oxygen concentration during fermentation. These culture conditions can be easily set by those skilled in the art.

In addition, the medium may contain a corresponding antibiotic in a case where a transformant expresses a useful additional trait, for example, in a case where a transformant contains a marker resistant to an antibiotic. By adding an antibiotic, a plasmid is stably retained. Examples of the antibiotic include, but are not limited to, ampicillin, kanamycin, chloramphenicol, tetracycline, erythromycin, streptomycin, and spectinomycin.

According to the present invention, it is possible to provide a recombinant polypeptide having an excellent carboxylic acid reducing activity, and a production method of an aliphatic compound using the recombinant polypeptide. Specifically, a mutation is introduced into a wild-type carboxylic acid reductase, such that a modified enzyme having a more improved enzyme activity as compared to the wild-type enzyme can be obtained. The target compound can be efficiently produced by using the present modified enzyme. In addition, various target compounds can be obtained by performing conversion using an additional enzyme in accordance with a desired target compound. Furthermore, since the modified enzyme according to the present invention has an improved enzyme activity, it is expected that the modified enzyme can be used for production of a target compound on an industrial scale.

Those skilled in the art given the above description can sufficiently implement the present invention. Hereinafter, examples are given for the purpose of further description, and the present invention is not limited to the examples.

### Examples

All the PCRs shown in the following Examples were performed using PrimeSTAR Max DNA Polymerase (Takara Bio Inc.). A transformation of Escherichia coli was performed using the calcium chloride method (see Genetic Engineering Experiment Note, first volume, YODOSHA CO., LTD., written by Takaaki Tamura). In the construction of the plasmid, an LB medium was used, and a necessary antibiotic was added thereto. As a reagent used in the present examples, a reagent manufactured by FUJIFILM Wako Pure Chemical Corporation was used unless otherwise specified. As a thermal cycler, C1000Touch touch thermal cycler manufactured by Bio-Rad Laboratories, Inc. was used.

The composition and the preparation method of the medium used in each example are as follows.

### (LB Medium)

20 g/L of an LB medium and Lenox (manufactured by Difco Laboratories, Inc.) were subjected to steam sterilization at 120°C for 20 minutes. If necessary, ampicillin at a final concentration of 100 mg/L and 1.5% Bacto agar (manufactured by Difco Laboratories, Inc.) were added.

### (SOC Medium)

First, 1 L of a solution of 20 g/L of bacto toryptone (manufactured by Difco Laboratories, Inc.), 5 g/L of bacto yeast extract (manufactured by Difco Laboratories, Inc.), 1 M NaCl, and 1 M KCl was prepared, steam sterilization was performed at 120°C for 20 minutes, and the solution was cooled to room temperature. Next, 10 ml of each of 1 M MgSO₄, 1 M MgCl₂, and 2 M glucose was sterilized by filtration, and added to the mixed solution described above.

### <1> Estimation of Carboxylic Acid Reductase Activity Center

In preparing a modified enzyme, amino acid residues to be considered to form an active center were estimated according to the following method. The protein crystal structure of the polypeptide of SEQ ID NO: 1 as a template has not been reported. Therefore, first, a crude model of the three-dimensional structure of the polypeptide of SEQ ID NO: 1 was prepared using swiss-model (https://swissmodel.expasy.org/, Swiss Institute of Bioinformatics) software. At this time, PDB entry registered as "5mst" was used as a template. The crude model was subjected to optimization of intramolecular interactions such as hydrogen bonding and van der Waals forces and structure optimization by Amber10 force field and Born solvent model using a Molecular Operating Environment (MOE) platform (manufactured by CCG ULC in Canada) to construct an initial model. Furthermore, molecular dynamics calculation was performed to obtain a stabilization model of the entire molecule. The molecular dynamics calculation was performed using Amber14, a package of force field parameters, and Generalized Born (GB) solvent model. Molecular motion during 1 ns (nanosecond) was simulated by calculation continuing the molecular state 500,000 times at 2 fs (femtosecond) intervals. By extending this calculation from 1 ns to 2 ns and 3 ns, it was estimated how the structure of the dynamically stable substrate-enzyme complex converged. Here, since force field parameters for calculating the dynamic structure of the substrate inside the enzyme were required, the transition state structure of the substrate (6-aminohexanoic acid or hexanedioic acid) in the enzymatic reaction was predicted using semi-empirical quantum mechanics calculation software MOPAC. Using Hamiltonian AM1, reaction path search and transition state optimization were performed, and further, a test by reference vibration analysis was performed. Structure optimization and charge calculation of this transition state were calculated using Hamiltonian of Hatree Fock 6/31G using Antechamber mounted on Gaussian.

The interaction between the substrate ligand bound to the enzyme and its surrounding amino acid residues was structurally optimized and evaluated from the binding data of the enzyme and the transition state substrate using MOE. At this time, the intramolecular interaction was optimized using a structure optimization algorithm Protonate 3D. Further, optimization of the whole complex was performed with Amber99 parameters in MOE. In addition, amino acid residues located within 5 Å around the substrate from the enzyme-substrate complex structure were identified by Visual Molecular Dynamics (VMD, University of Illinois) software.

### <2> Construction of Plasmid

A vector for gene expression was prepared as follows. First, a promoter sequence was inserted using BglII and EcoRV sites of the multiple cloning site of the plasmid pNFP-A51 (as FERM P-22182, deposited with Independent Administrative Institution, National Institute of Technology and Evaluation, International Patent Organism Depositary (IPOD) (address: Central 6, 1-1-1 Higashi, Tsukuba, Ibaraki, Japan) on October 25, 2011, International Deposition Accession No. FERM BP-11515), and a terminator sequence was inserted using XbaI and HindIII sites thereof, thereby constructing pSK000. At this time, three base pairs upstream of the EcoRV restriction enzyme recognition sequence were added to the promoter sequence terminal so that the EcoRV restriction enzyme site remained between the promoter and the terminator sequences, and it was used at the time of enzyme gene cloning described below. The promoter sequence and the terminator sequence are shown in Table 1. The primer sequences used are shown in Table 2. The pSK000 plasmid structure is illustrated in Fig. 6. The restriction enzymes used were manufactured by Takara Bio Inc.

**[Table 1]**

| | Gene sequence | SEQ ID NO: |
|---|---|---|
| Promoter | CTCAAGCCCAAAGGAAGAGTGAGGC GAGTCAGTCGCGTAATGCTTAGGCAC AGGATTGATTTGTCGCAATGATTGAC ACGATTCCGCTTGACGCTGCGTAAGG TTTTTGTAATTTTACAGGCAACCTTT TATTCACTAACAAATAGCTGGTGGAA | 3 |
| Terminator | AAAAAAAAAAGGTGCCGTGGAAAC ACGGCACCTTTTTTTTTTCTTTA | 6 |

**[Table 2]**

| Primer No. | Base sequence | SEQ ID NO: |
|---|---|---|
| 1 | ATGACTGAAACGATCTCCACAGCGG | 7 |
| 2 | CTACACCAGGCCCAACAGCTGAATA | 8 |
| 3 | ATGATCGAGACAATTTTGCCTGCTG | 9 |
| 4 | TCAGGCGTACGCGATCGCGG | 10 |
| 5 | ATGATACGCGAGCCTCCGGA | 11 |
| 6 | TTACGCTTCTTCGACACTTACTC | 12 |
| 7 | CTTCTTCTGCCACAAGTTGGCGACC | 13 |
| 8 | TCCGAAGTGCTCTTCGTGCCGC | 14 |
| 9 | ATGGGCAGCAGCCATCACCATC | 15 |
| 10 | GGTATATCTCCTTATTAAAGTTAAAC | 16 |
| 11 | TTTAATAAGGAGATATACCATGATACGCGAGCCTC | 17 |
| 12 | GGTGATGGCTGCTGCCCATTTACGCTTCTTCGACA | 18 |
| 13 | AGCTTGCGGCCGCATAATGCTTAAG | 19 |
| 14 | ATGTATATCTCCTTCTTATACTTAACTAATATAC | 20 |
| 15 | AGAAGGAGATATACATATGACTGAAACGATCTCCA | 21 |
| 16 | GGTGGCAGCAGCCTAGCTACACCAGGCCCAACA | 22 |

### (Preparation of Carboxylic Acid Reductase and Phosphopantetheinyl Transferase Expression Plasmid)

Polynucleotides encoding a carboxylic acid reductase and a phosphopantetheinyl transferase were synthesized using an artificial gene synthesis service of Eurofins Genomics K.K. Each enzyme was subjected to PCR amplification using a synthetic gene sequence as a template. A gene sequence encoding a carboxylic acid reductase was amplified under reaction conditions of 30 cycles of 98°C (10 sec), 55°C (5 sec), 72°C (20 sec), using primers 1 and 2 shown in Table 2. A gene sequence encoding a phosphopantetheinyl transferase was amplified under reaction conditions of 30 cycles of 98°C (10 sec), 55°C (5 sec), 72°C (10 sec), using primers 3 and 4.

Next, the resulting DNA fragment was phosphorylated with Mighty Cloning Reagent Set (manufactured by Takara Bio Inc.) and used as a DNA fragment to be cloned. Separately, the pSK000 plasmid was treated with an EcoRV restriction enzyme and then was treated with alkaline phosphatase (BAP, manufactured by Takara Bio Inc.). The DNA fragment and the vector fragment were ligated using Mighty Cloning Reagent Set (manufactured by Takara Bio Inc.) (16°C, overnight). An Escherichia coli JM109 strain was transformed with 1 µL of a ligation reaction solution. The plasmid was extracted from the appeared colonies and subjected to sequence analysis to prepare a plasmid into which a gene was inserted so that the enzyme protein was expressed.

### (Preparation of Aminotransferase Expression Plasmid)

A polynucleotide of an aminotransferase was synthesized using an artificial gene synthesis service of Eurofins Genomics K.K. A gene sequence was obtained from genebank gene ID7435770. PCR was performed under reaction conditions of 30 cycles of 98°C (10 sec), 55°C (5 sec), 72°C (10 sec), using a synthetic gene sequence as a template and primers 5 and 6 shown in Table 2. The resulting DNA fragment was ligated downstream of the promoter of the pSK000 vector by the method described above (16°C, overnight). An Escherichia coli JM109 strain was transformed with 1 µL of a ligation reaction solution. The plasmid was extracted from the appeared colonies and subjected to sequence analysis to obtain a plasmid into which a gene was inserted so that the enzyme protein was expressed.

### (Preparation of Point Mutation Introducing-Enzyme Expression Plasmid of Carboxylic Acid Oxidoreductase)

Next, a point mutation introduction comprehensive enzyme expression library of a carboxylic acid reductase gene sequence was prepared. First, 5 types of gene fragments of 200 base pairs in which 3 base pairs corresponding to a desired amino acid position were set to "NNK" were obtained using the artificial gene fragment synthesis service of Eurofins Genomics K.K. The sequences are shown in Table 3.

**[Table 3]**

| Mutation introduction site | Base sequence of synthetic fragment using comprehensive point mutation introduction | SEQ ID NO: |
|---|---|---|
| Trp 283 | CTTGTGGCAGAAGAAGNNKCTCACCGACGATGTGATTCCGTCC ATAGGCGTGAACTTCATGCCCATGAGCCACCTGGCGGGTCGCC TCACTCTCATGGGCACCCTTTCCGGTGGCGGAACCGCCTACTAC ATCGCTTCGAGCGATCTTTCGACTTTCTTCGAGGACATCGCGCT CATCCGCCCCTCCGAAGTGCTCTTCG | 23 |
| Leu 284 | CTTGTGGCAGAAGAAGTGGNNKACCGACGATGTGATTCCGTCC ATAGGCGTGAACTTCATGCCCATGAGCCACCTGGCGGGTCGCC TCACTCTCATGGGCACCCTTTCCGGTGGCGGAACCGCCTACTAC ATCGCTTCGAGCGATCTTTCGACTTTCTTCGAGGACATCGCGCT CATCCGCCCCTCCGAAGTGCTCTTCG | 24 |
| Ala 303 | CTTGTGGCAGAAGAAGTGGCTCACCGACGATGTGATTCCGTCCA TAGGCGTGAACTTCATGCCCATGAGCCACCTGNNKGGTCGCCT CACTCTCATGGGCACCCTTTCCGGTGGCGGAACCGCCTACTACA TCGCTTCGAGCGATCTTTCGACTTTCTTCGAGGACATCGCGCTC ATCCGCCCCTCCGAAGTGCTCTTCG | 25 |
| Leu 306 | CTTGTGGCAGAAGAAGTGGCTCACCGACGATGTGATTCCGTCCA TAGGCGTGAACTTCATGCCCATGAGCCACCTGGCGGGTCGCNN KACTCTCATGGGCACCCTTTCCGGTGGCGGAACCGCCTACTACA TCGCTTCGAGCGATCTTTCGACTTTCTTCGAGGACATCGCGCTC ATCCGCCCCTCCGAAGTGCTCTTCG | 26 |
| Leu 335 | CTTGTGGCAGAAGAAGTGGCTCACCGACGATGTGATTCCGTCCA TAGGCGTGAACTTCATGCCCATGAGCCACCTGGCGGGTCGCCT CACTCTCATGGGCACCCTTTCCGGTGGCGGAACCGCCTACTACA TCGCTTCGAGCGATCTTTCGACTTTCTTCGAGGACATCGCGNNK ATCCGCCCCTCCGAAGTGCTCTTCG | 27 |

Next, a vector fragment for cloning a point mutation introducing-enzyme gene fragment was amplified using 10 ng of the carboxylic acid reductase expression plasmid as a template. PCR was performed under reaction conditions of 30 cycles of 98°C (10 sec), 55°C (5 sec), 72°C (15 sec), using primers 7 and 8 shown in Table 2. The resulting vector fragment was linked to gene fragments of 200 base pairs using In-Fusion (registered trademark) HD Cloning Kit manufactured by Takara Bio Inc., thereby preparing a point mutation introducing-enzyme expression plasmid library.

### <3> Screening of Point Mutation Introducing-Enzyme Expression Plasmid Library

As a primary screen for modified carboxylic acid reductases, a comprehensive carboxylic acid reductase library into which the point mutation created in the [2] above was introduced was screened. First, an Escherichia coli JM109 strain was transformed by the point mutation introducing-enzyme expression plasmid library, and culture was performed on an agar medium at 30°C for 1 day. Similarly, for comparison, an Escherichia coli JM109 strain was transformed using a wild-type enzyme expression plasmid or an empty plasmid containing no enzyme gene, and the transformed strain was cultured in an agar medium at 30°C for 1 day. The appeared colonies were inoculated into an LB medium (deep well plate, 1 mL/well), and culture was performed at 30°C by shaking overnight (M·BR-1212FP plate shaker, manufactured by TAITEC CORPORATION). 30 µL of each culture solution was transferred to a microplate and diluted 10-fold, and OD600 values were measured and recorded (OD600) with Tecan Infinite 200 microplate reader. The deep well plate after culture was centrifuged at 2,456 xg for 10 minutes with Sorvall ST-8FL centrifuge (equipped with a plate rotor) manufactured by Thremo Fisher Scientific Inc. The supernatant was discarded, and the pellets were suspended in 50 µL of a 100 mM Tris-HCl buffer (pH = 8.0), and then 150 µL of a lysozyme solution was added (200 µL total, lysozyme at final concentration of 0.65 mg/mL, 1 mM EDTA). Incubation was performed at 30°C and 400 rpm for 30 minutes and centrifuged at 2,546 xg for 10 minutes using Deepwell Maximizer tabletop shaker manufactured by TAITEC CORPORATION. 50 uL of the supernatant was transferred to a 96 well microplate and the substrate solution pre-warmed at 30°C was added (250 µL total, final concentration 0.2 mM NADPH, 0.5 mM ATP, 10 mM disodium adipate, 10 mM MgCl₂, 100 mM HEPES potassium buffer pH = 8.0). An absorbance at 340 nm was serially measured for 15 minutes using Tecan Infinite 200 microplate reader. A decrease amount of the absorbance at 340 nm per unit time was calculated, and the calculated value was divided by the OD600 value. As a result of comparing the obtained values between the wild-type enzyme and the mutant enzyme, a plurality of mutants showing an activity exceeding that of the wild-type enzyme were obtained. Passage colonies in the well in which a rate of decrease in the absorbance at 340 nm exceeded that of the wild-type were cultured in an LB medium, the plasmid was extracted, and sequence analysis was performed. For each modified enzyme obtained from the screening, the mutation point and the relative enzyme activity with respect to the activity of the wild-type carboxylic acid reductase are shown in Table 4.

**[Table 4]**

| Table 4: Specific activity of each modified enzyme | | | | | | | | | |
|---|---|---|---|---|---|---|---|---|---|
| | | Specific activity of each mutant when wild-type is used as 1 | | | | | | | |
| | | Amino acid introduced into mutant | | | | | | | |
| | | Lys | Arg | Thr | Val | Met | Phe | Tyr | Cys |
| Mutation site in enzyme | Trp 283 | 1.22 | 1.26 | 1.05 | | | | | |
| | Leu 284 | | 1.08 | 1.05 | 1.24 | | | | |
| | Ala 303 | | | | | 1.51 | | | 1.36 |
| | Leu 306 | 1.14 | 1.12 | | | | | | |
| | Leu 335 | | 1.27 | | | 1.22 | 1.20 | 1.25 | |

### <4> Construction of Double Mutation-Introducing Carboxylic Acid Reductase and Evaluation of Activity against Various Substrates

### (Preparation of Double Mutation-Introducing Carboxylic Acid Reductase Expression Plasmid)

Based on the amino acid mutation information obtained in the screening to improve the carboxylic acid reductase activity, a mutant expression plasmid in which two mutation points were combined was prepared, and an enzyme activity comparison was performed. A second mutation introduction into the carboxylic acid reductase point mutation gene sequence was performed under reaction conditions of 30 cycles of 98°C (10 sec), 55°C (5 sec), 72°C (10 sec), using InversePCR method. The resulting DNA fragment was phosphorylated with Mighty Cloning Reagent Set (manufactured by Takara Bio Inc.) and self-ligated (16°C, 20 minutes). An Escherichia coli JM109 strain was transformed with 1 µL of a ligation reaction solution. Plasmids were extracted from the appeared colonies and subjected to sequence analysis to confirm that the target mutation was introduced.

### (Synthesis of 6-Oxohexanoic Acid)

6-Oxohexanoic acid was synthesized with reference to the literature (Turk et.al. Metabolic Engineering toward Sustainable Production of Nylon-, ACS Synthetic Biology, ACS Publications, 2016, vol. 5, pp 65-73). 1 g of methyl 6-oxohexanoate manufactured by Toronto Research Chemicals Inc. was added to water to obtain 10% w/w%. While stirring with a magnetic stirrer, 8 N NaOH was added to adjust the pH to 14. The mixed solution was stirred at room temperature for 24 hours as it was, and 5 N HCl was added to neutralize the solution to a pH of 7. The solution was dispensed into a 1.5 mL tube by 100 µL, and concentrated for 2 hours using CVE-3110 centrifugal evaporator manufactured by Tokyo Rikakikai Co., Ltd. Freezing was performed at -80°C for 3 hours, and then drying was performed in a vacuum dryer overnight.

### (Evaluation of Activity of Double Mutation-Introduced Carboxylic Acid Reductase)

An Escherichia coli JM109 strain was transformed with a double mutation-introducing enzyme expression plasmid. Similarly, for comparison, the Escherichia coli JM109 strain was transformed using either a wild-type enzyme expression plasmid or an empty plasmid containing no enzyme gene, and the transformed strain was cultured in an agar medium at 30°C for 1 day. The appeared colonies were inoculated into an LB medium (15 m tube, liquid volume 2 mL), and culture was performed at 30°C by shaking overnight. The remaining culture solution was transferred to a 2 mL tube, and centrifugation was performed at 8,000 rpm for 3 minutes with a Centrifuge 5424R desktop micro centrifuge manufactured by Eppendorf SE. The supernatant was discarded, the pellets were suspended in 200 µL of a 100 mM Tris-HCl buffer (pH = 8.0), and then bacterial cells were disrupted with an ultrasonic crusher. Ultrasonic disruption was performed on ice at the output of the memory 3 using a protocol of oscillation for 30 seconds, pause for 30 seconds, and oscillation for 30 seconds (UR-21P manufactured by TOMY COMPANY, LTD.). The disrupted solution was centrifuged at 13,200 rpm for 15 minutes, and the supernatant was used as an enzyme solution. The enzyme solution was diluted 2-fold with a 100mM Tris-HCl buffer (pH = 8.0), 50 µL of the diluted enzyme solution was transferred to a 96 well microplate, and the substrate solution pre-warmed at 30°C was added (250 µL total, final concentration 0.2 mM NADPH, 0.5 mM ATP, 10 mM MgCl₂, 100 mM HEPES potassium buffer pH = 8.0). At this time, as a substrate, any one of 1 mM disodium adipate, 1 mM 6-oxohexanoic acid, 10 mM 6-aminocaproic acid, and 0.1 mM 6-hydroxycaproic acid was used. 6-Hydroxycaproic acid was purchased from AccuStandard, Inc. An absorbance at 340 nm was serially measured for 15 minutes using Tecan Infinite 200 microplate reader. 5 µL of the assay solution after the reaction was sampled, and the total protein concentration was calculated on the basis of the Bradford method using a protein assay concentrated dye reagent manufactured by Bio-Rad Laboratories, Inc.

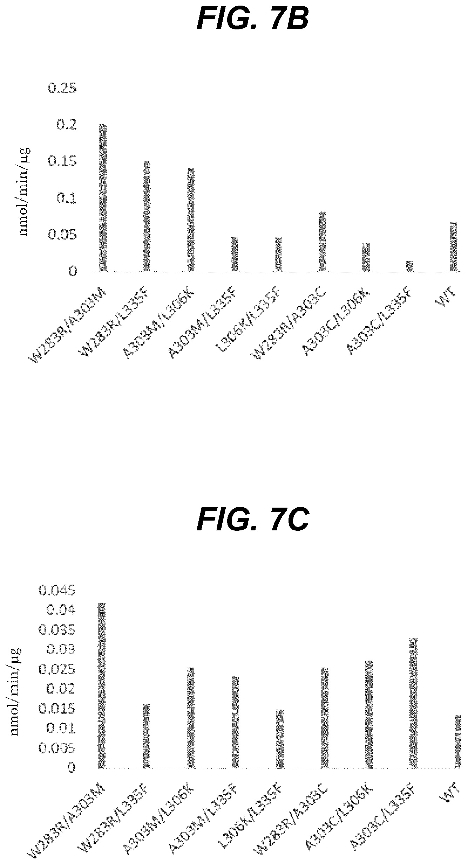

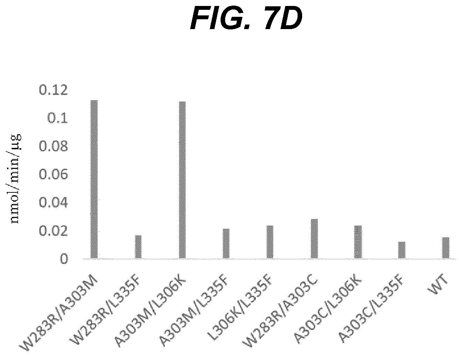

Separately, 5 µL of the enzyme solution was fractionated as a sample for SDS-PAGE, and mixed with 5 µL of a 2x laemmuli sample buffer (5% 2-mercapto-1,3-propanediol) manufactured by Bio-Rad Laboratories, Inc. The solution was heated at 100°C for 20 minutes, and then the heated solution was migrated at 200 V for 30 minutes using Mini-PROTEAN TGX Gel (Any kD) manufactured by Bio-Rad Laboratories, Inc. and Mini-PROTEAN Tetra Cell SDS-PAGE electrophoresis tank. The gel after electrophoresis was dyed for 30 minutes with Coomassie stain solution manufactured by Bio-Safe, and decolorized with distilled water for 2 hours. The decolorized gel was imaged with Gel DocEZ system manufactured by Bio-Rad Laboratories, Inc., and a proportion of carboxylic acid reductase bands in the total protein bands of each lane was calculated using Image lab software. The total protein concentration was multiplied by the carboxylic acid reductase band proportion to obtain the carboxylic acid reductase concentration in the assay solution. Furthermore, the NADPH consumption rate was calculated from the change in absorbance at 340 nm, and the NADPH consumption rate per unit enzyme protein was calculated. All values are mean values of two independent culture samples. Figs. 7A, 7B, 7C, and 7D illustrate the double mutation-introduced carboxylic acid reductase evaluated and the activity against each substrate. The enzyme containing double mutantion in which the amino acid at position 283 was mutated to arginine and the amino acid at position 303 was mutated to methionine showed an enhanced activity against all of the substrates.

### <5> Change in Enzymatic Properties of Double Mutation-Introduced Carboxylic Acid Reductase

The enzymatic properties of the double mutation enzyme and the wild-type enzyme were compared. An Escherichia coli JM109 strain was transformed using any one of a wild-type enzyme expression plasmid, a mutant enzyme expression plasmid set forth in SEQ ID NO: 3, and an empty plasmid having no enzyme gene. The appeared colonies were inoculated into an LB medium (15 mL tube, liquid volume 2 mL), and culture was performed at 30°C by shaking overnight. 30 µL of each culture solution was diluted 10-fold, and the OD600 value was recorded. The remaining culture solution was transferred to a 2 mL tube, and centrifugation was performed at 8,000 rpm for 3 minutes with a Centrifuge 5424R tabletop micro centrifuge manufactured by Eppendorf SE. The supernatant was discarded, the pellets were suspended in a 100 mM Tris-HCl buffer (pH = 8.0) so that OD600 = 30, and then bacterial cells were disrupted with an ultrasonic crusher. The disrupted solution was centrifuged at 13,200 rpm for 15 minutes, and the supernatant was used as an enzyme solution. The enzyme solution was diluted 3-fold with a 100 mM Tris-HCl buffer (pH = 8.0), 50 µL of the diluted enzyme solution was transferred to a 96 well microplate, and the substrate solution pre-warmed at 30°C was added (250 µL total, final concentration 0.2 mM NADPH, 0.5 mM ATP, 10 mM MgCl₂, 100 mM HEPES potassium buffer pH = 8.0). For the experiment of a wild-type enzyme, disodium adipate was used at a concentration of any of 50 mM, 25 mM, 12.5 mM, 6.25 mM, and 3.125 mM. For the experiment of a mutant enzyme, disodium adipate was used at a concentration of any of 3 mM, 1.5 mM, 0.75 mM, 0.375 mM, and 0.1875 mM. An absorbance at 340 nm was serially measured for 60 minutes using Tecan Infinite 200 microplate reader. 5 µL of the assay solution after the reaction was sampled, and the total protein concentration was calculated on the basis of the Bradford method using a protein assay concentrated dye reagent manufactured by Bio-Rad Laboratories, Inc.

Separately, 5 µL of the enzyme solution was fractionated as a sample for SDS-PAGE, and mixed with 5 µL of a 2x laemmuli sample buffer (5% 2-mercapto-1,3-propanediol) manufactured by Bio-Rad Laboratories, Inc. The solution was heated at 100°C for 20 minutes, and then the heated solution was subjected to electrophoresis at 200 V for 30 minutes using Mini-PROTEAN TGX Gel (Any kD) manufactured by Bio-Rad Laboratories, Inc. and Mini-PROTEAN Tetra Cell SDS-PAGE electrophoresis tank. The gel after electrophoresis was dyed for 30 minutes with Coomassie stain solution manufactured by Bio-Safe, and decolorized with distilled water for 2 hours. The decolorized gel was imaged with Gel DocEZ system manufactured by Bio-Rad Laboratories, Inc., and a proportion of carboxylic acid reductase bands in the total protein bands of each lane was calculated using Image lab software. The total protein concentration was multiplied by the carboxylic acid reductase band proportion to obtain the carboxylic acid reductase concentration in the assay solution. Furthermore, the NADPH consumption rate was calculated from the change in absorbance at 340 nm, and the NADPH consumption rate per unit enzyme protein was calculated. The inverse of the substrate concentration and the inverse of the NADPH consumption rate were plotted, and Km and Vmax were calculated from the intersection with the x-axis and the intersection with the y-axis, respectively. Each numerical value is shown in the following table. The numerical values are mean values of two independent studies. Km of the mutant enzyme for adipic acid was 1/20 or less that of the wild-type enzyme, was 1/17 compared to the existing variant carboxylic acid reductases, that is, L342 and G418E mutants (Fedrchuk et. al. One-pot Biocatalytic Transformation of Adipic Acid to 6-aminocaproic Acid and 1,6-hexamethylenediamine Using Carboxylic Acid Reductases and Transaminases. J. Am. Chem. Soc., 2020, 142, 2, pp. 1038-1048 (Non Patent Literature 6)), and was improved compared to the wild-type enzyme.

**[Table 5]**

| Table 5: Comparison of enzymatic properties of wild-type carboxylic acid reductase, modified enzyme (SEQ ID NO: 3), and existing modified enzymes | | | |
|---|---|---|---|
| | Km (mmol/L) | Vₘₐₓ (umol/L/min) | Kcat (sec⁻¹) |
| Comparative Example (wild-type, SEQ ID NO: 1) | 23.3 | 18.1 | 1.68 |
| Example (W283R/A303M mutant, SEQ ID NO: 3) | 1.06 | 24.8 | 3.53 |
| Comparative Example (L342E mutant, literature value) | 56.6+/-5.0 | Not shown | 2.72+/-0.09 |
| Comparative Example (G418E mutant, literature value) | 18.7+/-2.5 | Not shown | 4.25+-/0.26 |

### <6> Synthesis of Chemical Products Using Double Mutation-Introduced Carboxylic Acid Reductase

When an enzyme solution was prepared using Escherichia coli, an alcohol dehydrogenase ADH gene-disrupted strain was used as an Escherichia coli strain expressing an enzyme in order to avoid contamination with an alcohol dehydrogenase unique to Escherichia coli. A method for preparing an ADH gene disruption plasmid will be described below.

### (Preparation of ADH Gene Disruption Plasmid)

Disruption of an ADH gene was performed by a homologous recombination method using pHAK1 (as NITE P-02919, deposited with biotechnology division of National Institute of Technology and Evaluation (NITE), Patent Microorganisms Depositary (NPMD) (address: #122, 2-5-8, Kazusakamatari, Kisarazu-shi, Chiba, Japan) on March 18, 2019, International Accession No: NITE BP-02919). pHAK1 includes a temperature-sensitive mutant repA gene, a kanamycin resistant gene, and a levansucrase gene SacB derived from Bacillus subtilis. The levansucrase gene lethally acts on a host microorganism in the presence of sucrose. The PCR fragment was amplified using PrimeSTAR Max DNA Polymerase (trade name, manufactured by Takara Bio Inc.), and the plasmid was prepared using an Escherichia coli HST08 strain. Using the genomic DNA of the Escherichia coli BL21 (DE3) strain as a template, a PCR product containing an upstream region, a coding region, and a downstream region of a disruption target gene was obtained. The combinations of the target gene and the primer sequence are shown in Table 6.

**[Table 6]**

| Target gene | Forward primer | | | Reverse primer | | |
|---|---|---|---|---|---|---|
| | Primer No. | Base sequence | SEQ ID NO: | Primer No. | Base sequence | SEQ ID NO: |
| yqhD | 17 | TTGTTGAATAAATCGT CTGTTTGCCGAGAAT ACGCG | 28 | 18 | TATCGCATGCAGA TCTGCGCTAATGG GCTCCAGGA | 29 |
| fucO | 19 | TTGTTGAATAAATCGC GCGCCAGCGCTGGCT GTTT | 30 | 20 | TATCGCATGCAGA TCCCGCCAATGCC GGAAGAGTT | 31 |
| adhP | 21 | TTGTTGAATAAATCGC GATCGTGATGCCGCT GTCT | 32 | 22 | TATCGCATGCAGA TCGACAACGTAGG CTTTGTTCA | 33 |
| eutG | 23 | TTGTTGAATAAATCGC GCCATCTCGACACTCT TGA | 34 | 24 | TATCGCATGCAGA TCGCGAACATCGA TGGGTTAGC | 35 |
| ybbO | 25 | TTGTTGAATAAATCGT GGCATTTGCCCTTCCT GTT | 36 | 26 | TATCGCATGCAGA TCGTCCTGATCCT GCAACGGAA | 37 |
| ahr | 27 | TTGTTGAATAAATCGC TCATAACGGTACTGCA AAC | 38 | 28 | TATCGCATGCAGA TCTCGCAGCAGGT AAGATGATT | 39 |
| yahK | 29 | TTGTTGAATAAATCGA TATTCGTCCTAACGAA CAG | 40 | 30 | TATCGCATGCAGA TCTTTTTGATTTTC AAGTATGT | 41 |

Next, the present PCR product was inserted into the pHAK1 plasmid fragment amplified using primers 31 and 32 using In-Fusion (registered trademark) HD cloning kit (trade name, manufactured by Clontech Laboratories, Inc.) and the PCR product was circularized. The sequences of the primers 31 and 32 are shown in Table 7.

**[Table 7]**

| Primer No. | Base sequence | SEQ ID NO: |
|---|---|---|
| 31 | GATCTGCATGCGATATCTCTAGAACGCGTAAGCTT | 42 |
| 32 | TCTCGAGCCGATITATTCAACAAAGCCGC | 43 |

PCR was performed using the resulting pHAK1 plasmid into which the DNA fragment containing the upstream region, the coding region, and the downstream region of the disruption target gene was inserted, as a template, and using primers shown in Table 8, thereby obtaining a plasmid fragment in which the coding region of the disruption target gene was partially or entirely removed.

**[Table 8]**

| Target gene | Forward primer | | | Reverse primer | | |
|---|---|---|---|---|---|---|
| | Primer No. | Base sequence | SEQ ID NO: | Primer No. | Base sequence | SEQ ID NO: |
| yqhD | 33 | ACTTTCGTTTTCGG GCATTTCGTCC | 44 | 34 | CCAATATGAGGGCAGAGA ACGATC | 45 |
| fucO | 35 | ATGCGCTGATGTGA TAATGC | 46 | 36 | CCTTCTCCTTGTTGCTTTA | 47 |
| adhP | 37 | GAGGCCTTTGCTGC GACTGC | 48 | 38 | AGTTCCTCCTTTTCGGATG AT | 49 |
| eutG | 39 | ATGCCGGATGCGA CGCTT | 50 | 40 | TCATTTTGCATATAGCCCC T | 51 |
| ybbO | 41 | TGACCTGGGCAGT AATGGTG | 52 | 42 | CAGGATCTCCGTTGCTTTA TGAGTC | 53 |
| ahr | 43 | CGTGGTGTTGAAA GCCGATTATTG | 54 | 44 | CATAAACTTCCAGTTCTCC GCCC | 55 |
| yahK | 45 | TCGCACACTAACAG ACTGAA | 56 | 46 | TGTGTTTACTCCTGATTAG C | 57 |

The resulting plasmid fragment was circularized by terminal phosphorylation and self-ligation to obtain a plasmid for disrupting a gene.

### (Construction of ADH Gene-Disrupted Escherichia Coli Strain)

An Escherichia coli BL21 (DE3) strain was transformed with a plasmid for destruction of a desired gene by a calcium chloride method and then applied to an LB agar medium containing 100 mg/L of kanamycin sulfate, and culture was performed at 30°C overnight, thereby obtaining a transformant. The present transformant was inoculated into 1 mL of an LB medium containing 100 mg/L of kanamycin sulfate with a platinum loop, and shaking culture was performed at 30°C. The resulting culture medium was applied to an LB agar medium containing 100 mg/L of kanamycin sulfate, and culture was performed at 42°C overnight. In the resulting colony, a plasmid was inserted into the genome by single crossover. The colony was inoculated into 1 mL of an LB liquid medium with a platinum loop, and shaking culture was performed at 30°C. The resulting culture medium was applied to an LB agar medium containing 10% sucrose, and culture was performed overnight. Disruption of a desired gene in the resulting colony was confirmed by colony direct PCR using the primer set shown in Table 9. An ADH-deficient Escherichia coli in which the ADH7 gene was disrupted was constructed from the above operation.

**[Table 9]**

| Target gene | Forward primer | | | Reverse primer | | |
|---|---|---|---|---|---|---|
| | Primer No. | Base sequence | SEQ ID NO: | Primer No. | Base sequence | SEQ ID NO: |
| yqhD | 47 | TATTCTCAATCCGTTT CAGCACGCG | 58 | 48 | TTCGGGATCACCACCAGGCCG | 59 |
| fucO | 49 | CGGAAATGGACGAAC AGTGG | 60 | 50 | CGTCATCAGCGTTTACCAGAT T | 61 |
| adhP | 51 | GCATAAACACTGTCCG CGTC | 52 | 52 | GAAATCGAGAAGGCAGAAGC GAAA | 63 |
| eutG | 53 | GGTGCGGTCACCATT GTTCG | 64 | 54 | CATATCGCACGCCAGCAGTG | 65 |
| ybbO | 55 | GGTGAGGATGGAGAG TTCATG | 66 | 56 | CAGTTCGATTTGCGCCACCAG | 67 |
| ahr | 57 | TCCGCTAGTGTGATTT CAGG | 68 | 58 | GAAATTATTATGCCGCCAGGC GT | 69 |
| yahK | 59 | TTATGGTCTGGGCGAC ATGC | 70 | 60 | GCATCATCCTGGTCATATACC C | 71 |

### (Preparation of Enzyme)

The ADH-deficient Escherichia coli strain was transformed using any one of a wild-type carboxylic acid reductase expression plasmid, a modified enzyme expression plasmid set forth in SEQ ID NO: 3, an aminotransferase expression plasmid, and an empty plasmid having no enzyme gene.

The colonies were inoculated into an LB medium (15 m tube, liquid volume 2 mL), and culture was performed at 30°C by shaking overnight. 30 µL of each culture solution was diluted 10-fold, and OD600 values were measured and recorded with Tecan Infinite 200 microplate reader. The remaining culture solution was transferred to a 2 mL tube, and centrifugation was performed at 8,000 rpm for 3 minutes with a Centrifuge 5424R tabletop micro centrifuge manufactured by Eppendorf SE. The supernatant was discarded, the pellets were re-suspended in a 100 mM Tris-HCl buffer (pH = 8.0) so that OD600 = 30, and then bacterial cells were disrupted with an ultrasonic crusher. The disrupted solution was centrifuged at 13,200 rpm for 15 minutes, and the supernatant was used as an enzyme solution. The enzymatic reaction was performed in a 1.5 mL tube. 50 µL each of enzyme solutions containing a carboxylic acid reductase and an aminotransferase was added to the reaction solution. The composition of the reaction solution is as follows.

Reaction conditions 1: final concentration 2 mM disodium adipate, 5 mM sodium glutamate, 5 mM NADPH, 5 mM ATP, 10 mM MgCl₂, 0.05 mM pyridoxal phosphate, 100 mM HEPES potassium buffer pH = 8.0, total volume 1,000 µL.

Reaction conditions 2: final concentration 10 mM 6-aminocaproic acid, 20 mM sodium glutamate, 20 mM NADPH, 20 mM ATP, 10 mM MgCl₂, 0.05 mM pyridoxal phosphate, 100 mM HEPES potassium buffer pH = 8.0, total volume 1,000 µL.

The prepared tube was shaken at 30°C and 1,000 rpm for 4 hours with a tabletop shaker (M·BR-022UP) manufactured by TAITEC CORPORATION. The sample after the reaction was diluted 5-fold with 0.5% formic acid and analyzed by the analysis method shown in Table 10.

**[Table 10]**

| Analysis of carboxylic acid and alcohol | |
|---|---|
| Column | Shim-Pack SCR(H) + Shim-pack SCR-101H 7.9 × 300 (manufactured by Shimadzu Corporation) |
| Column temperature | 40°C |
| Eluant | Phosphoric acid aqueous solution (pH 2.3) 0.1 wt% |
| Flow rate | 1 mL/min |
| Detector | RI + UV 210 nm |
| Injection amount | 50 uL |
| | |

| Analysis of amino alcohol | |
|---|---|
| Column | Shodex IC YS-G + YS-50 (4.6 mm × 125 mm) (manufactured by Showa Denko K.K.) |
| Column temperature | 40°C |
| Eluant | 6 mM MSA |
| Flow rate | 1 mL/min |
| Detector | RI |
| Injection amount | 10 uL |
| | |

| Analysis of amine | |
|---|---|
| Column | Dionex IonPac CG19 +CS19 (2 mm) (manufactured by Thermo Fisher Scientific Inc.) |
| Column temperature | 30°C |
| Eluant | 9 mM MSA → 70 mM MSA (10 min) |
| | → hold for 1 min |
| Flow rate | 0.35 mL/min |
| Detector | Suppressed Conductivity (CSRS 300) |
| Injection amount | 5 uL |

Tables 11-1 and 11-2 show the analysis results of the concentration (µM) of hexamethylenediamine HMD produced when the substrate compound is enzymatically treated with a carboxylic acid reductase and an aminotransferase. The respective values are mean values of two independent assays. In Tables 11-1 and 11-2, the substrates used are adipic acid (Table 11-1) and 6-aminocaproic acid (Table 11-2), respectively. In the tables, "CAR" indicates a carboxylic acid reductase, "AT" indicates an aminotransferase, and "blank vector" indicates that a disrupted product of bacterial cells transformed with an empty vector having no enzyme gene sequence is used. The "variant CAR" used here is a modified enzyme consisting of an amino acid sequence set forth in SEQ ID NO: 3.

**[Table 11-1]**

| Reaction condition 1 Sample | HMD (µM) |
|---|---|
| AT + wild-type CAR (Comparative Example) | 8.63 |
| AT + variant CAR (Example) | 403 |
| Only AT (Comparative Example) | 0.959 |
| Only wild-type CAR (Comparative Example) | N.D. |
| Only variant CAR (Comparative Example) | N.D. |
| Blank vector (Comparative Example) | N.D. |

As shown in Table 11-1, production of HMD from adipic acid was not observed in the sample in which neither a carboxylic acid reductase nor an aminotransferase was added and the sample in which only a carboxylic acid reductase was added. In a case where both a wild-type carboxylic acid reductase and an aminotransferase were added, slight HMD production was observed, but in comparison, in a case where a variant carboxylic acid reductase and an aminotransferase were used, a 50-fold increase in HMD production was observed. It was found that the use of the present mutant enzyme can contribute to the production of HMD even without a coenzyme regeneration system.

**[Table 11-2]**

| Reaction condition 2 Sample | HMD (µM) |
|---|---|
| AT + wild-type CAR (Comparative Example) | 16.1 |
| AT + variant CAR (Example) | 81.4 |
| Only AT (Comparative Example) | 4.96 |
| Only wild-type CAR (Comparative Example) | N.D. |
| Only variant CAR (Comparative Example) | 19.7 |
| Blank vector (Comparative Example) | N.D. |

As shown in Table 11-2, production of HMD from 6-aminocaproic acid was not observed in the sample in which neither a carboxylic acid reductase nor an aminotransferase was added and the sample in which only a wild-type carboxylic acid reductase was added. In a case where a wild-type carboxylic acid reductase and an aminotransferase were added together, HMD production was observed. Interestingly, in the sample to which only a variant carboxylic acid reductase was added, more efficient generation of HMD was observed than in the case where a wild-type carboxylic acid reductase and an aminotransferase were added together. It can be presumed that 6-aminocaproic acid was more efficiently reduced by the variant carboxylic acid reductase, and further converted into HMD by the aminotransferase intrinsic to Escherichia coli. In addition, the highest HMD concentration was observed in the sample to which both a variant carboxylic acid reductase and an aminotransferase were added.

### <7> Synthesis of Chemical Products Using Double Mutation-Introduced Carboxylic Acid Reductase Expression Bacteria

### (Preparation of Carboxylic Acid Reductase and Aminotransferase Co-Expression Plasmid)

pACYCDuet (manufactured by Merck KGaA) was used as a backbone of the co-expression plasmid. First, in order to insert ygjg into a multiple cloning site 1, PCR was performed using primer Nos. 9 and 10 and pACYCDuet as a template to prepare vector fragments. Next, PCR was performed using primer Nos. 11 and 12 and the plasmid obtained by cloning the aminotransferase as a template to prepare ygjg gene fragments. The resulting gene fragment was ligated using In-Fusion (registered trademark) HD Cloning Kit manufactured by Takara Bio Inc. Next, in order to insert a carboxylic acid reductase into a multiple cloning site 2, PCR was performed using the ygjg-containing pACYC plasmid obtained in the above operation as a template and primer Nos. 13 and 14 to obtain fragments to be used as a vector. As the PCR reaction conditions, 30 cycles of 98°C (10 sec), 55°C (5 sec), 72°C (30 sec) were used.

Next, PCR was performed using a plasmid obtained by cloning a wild-type carboxylic acid reductase or a double mutation carboxylic acid reductase as a template and using primer Nos. 15 and 16. As the PCR reaction conditions, 30 cycles of 98°C (10 sec), 55°C (5 sec), 72°C (20 sec) were used. A vector fragment and a PCR fragment containing a wild-type or variant carboxylic acid reductase were ligated using In-Fusion (registered trademark) HD Cloning Kit manufactured by Takara Bio Inc., thereby preparing a two-gene co-expression plasmid.

### (Culture Evaluation)

As an Escherichia coli strain for expressing an enzyme, the ADH-disrupted strain prepared in the <6> above was used. The strain was transformed with a two-gene co-expression plasmid or a pACYC or pACYC plasmid in which ygjg alone was cloned. The colonies were inoculated into an LB medium (15 m tube, liquid volume 1 mL), and culture was performed at 37°C by shaking for 3 hours. Next, 5% of the colonies were inoculated into an SOC medium containing 10 mM adipic acid and culture was performed at 30°C overnight (15 ml tube, liquid volume 2 ml). 30 uL of each culture solution was diluted 10-fold, and OD600 values were measured and recorded using Tecan Infinite 200 microplate reader. The remaining culture solution was transferred to a 2 mL tube, and centrifugation was performed at 8,000 rpm for 3 minutes with a Centrifuge 5424R tabletop micro centrifuge manufactured by Eppendorf SE. The supernatant was diluted 5-fold with 0.5% formic acid and analyzed by the analysis method shown in Table 10.

Table 12 shows the concentration (µM) of HMD detected in the supernatant after culturing a strain expressing a wild-type or modified carboxylic acid reductase and an aminotransferase for a certain period of time in a medium containing adipic acid. In the table, "modified CAR1" is a recombinant polypeptide having an amino acid sequence set forth in SEQ ID NO: 3 (modified carboxylic acid reductase 1), and "modified CAR2" is a recombinant polypeptide having an amino acid sequence set forth in SEQ ID NO: 4 (modified carboxylic acid reductase 2). The respective values are mean values of two independent assays.

**[Table 12]**

| Introduced carboxylic acid reductase | HMD (µM) |
|---|---|
| Modified CAR 1 (Example) | 116.1 |
| Modified CAR 2 (Example) | 104.4 |
| Wild-type (Comparative Example) | 72.1 |
| Blank vector (Comparative Example) | N.D. |

As shown in Table 12, production of HMD from adipic acid was not observed in the culture sample of the strain transformed with an empty vector that did not express a carboxylic acid reductase and an aminotransferase. Production of HMD was observed in the culture sample of the strain co-expressing a wild-type carboxylic acid reductase and an aminotransferase. In comparison to this, a 1.4- to 1.6-fold increase in production of HMD was observed in a case where a variant carboxylic acid reductase and an aminotransferase were used. It was found that intracellular conversion from adipic acid to HMD was promoted by using a variant carboxylic acid reductase.

### Industrial Applicability

The present invention can be used for efficient fermentation production including a carboxylic acid reduction reaction, and is expected to be applied to produce a target compound on an industrial scale.

## Claims

1. A recombinant polypeptide comprising:
(a) an amino acid sequence A having a sequence identity of 60% or higher, 65% or higher, 70% or higher, 75% or higher, 80% or higher, 85% or higher, 88% or higher, 90% or higher, 95% or higher, 97% or higher, 98% or higher, or 99% or higher with an amino acid sequence set forth in SEQ ID NO: 1;
(b) a substitution of at least one amino acid at a position corresponding to a substrate-binding site of a polypeptide having an amino acid sequence set forth in SEQ ID NO: 1 in the amino acid sequence A; and
(c) a carboxylic acid reducing activity.

2. The recombinant polypeptide according to claim 1, wherein the position corresponding to the substrate-binding site of the polypeptide having the amino acid sequence set forth in SEQ ID NO: 1 in (b) is a position corresponding to positions 283, 284, 298, 303, 306, 335, 512, and 926 based on the amino acid sequence set forth in SEQ ID NO: 1.

3. The recombinant polypeptide according to claim 1 or 2, wherein (b) in the amino acid sequence A, at least two amino acids at the position corresponding to the substrate-binding site of the polypeptide having the amino acid sequence set forth in SEQ ID NO: 1 are substituted.

4. The recombinant polypeptide according to any one of claims 1 to 3, wherein (b) in the amino acid sequence A, based on the amino acid sequence set forth in SEQ ID NO: 1,
· positions corresponding to positions 283 and 303 are substituted,
· positions corresponding to positions 283 and 335 are substituted,
· positions corresponding to positions 303 and 306 are substituted,
· positions corresponding to positions 303 and 335 are substituted,
· positions corresponding to positions 306 and 335 are substituted, or
· positions corresponding to positions 283 and 303 are substituted.

5. The recombinant polypeptide according to any one of claims 1 to 4, wherein (b) in the amino acid sequence A, at least one of the following (i) to (v) is satisfied based on the amino acid sequence set forth in SEQ ID NO: 1:
(i) an amino acid residue at a position corresponding to position 283 is substituted with any of arginine, threonine, and lysine;
(ii) an amino acid residue at a position corresponding to position 284 is substituted with any of arginine, threonine, and valine;
(iii) an amino acid residue at a position corresponding to position 303 is substituted with any of cysteine and methionine;
(iv) an amino acid residue at a position corresponding to position 306 is substituted with any of arginine and lysine; and
(v) an amino acid residue at a position corresponding to position 335 is substituted with any of arginine, tyrosine, phenylalanine, and methionine.

6. The recombinant polypeptide according to any one of claims 1 to 5, wherein (b) in the amino acid sequence A, any one of the following (xi) to (xviii) is satisfied based on the amino acid sequence set forth in SEQ ID NO: 1:
(xi) an amino acid residue at a position corresponding to position 283 is arginine and an amino acid at a position corresponding to position 303 is methionine;
(xii) an amino acid residue at a position corresponding to position 283 is arginine and an amino acid at a position corresponding to position 303 is cysteine;
(xiii) an amino acid residue at a position corresponding to position 283 is arginine and an amino acid at a position corresponding to position 335 is phenylalanine;
(xiv) an amino acid residue at a position corresponding to position 303 is methionine and an amino acid at a position corresponding to position 306 is lysine;
(xv) an amino acid residue at a position corresponding to position 303 is methionine and an amino acid at a position corresponding to position 335 is phenylalanine;
(xvi) an amino acid residue at a position corresponding to position 303 is cysteine and an amino acid at a position corresponding to position 306 is lysine;
(xvii) an amino acid residue at a position corresponding to position 303 is cysteine and an amino acid at a position corresponding to position 335 is phenylalanine; and
(xviii) an amino acid residue at a position corresponding to position 306 is lysine and an amino acid at a position corresponding to position 335 is phenylalanine.

7. The recombinant polypeptide according to any one of claims 1 to 6, wherein the carboxylic acid reducing activity of (c) is improved compared to a carboxylic acid reducing activity of the polypeptide having the amino acid sequence set forth in SEQ ID NO: 1.

8. The recombinant polypeptide according to any one of claims 1 to 7, wherein the recombinant polypeptide is derived from a microorganism of a genus selected from the group consisting of the genus Mycobacterium, the genus Nocardia, the genus Neurospora, and the genus Segniliparus.

9. The recombinant polypeptide according to any one of claims 1 to 8, wherein the recombinant polypeptide is derived from a microorganism of the genus Mycobacterium.

10. The recombinant polypeptide according to any one of claims 1 to 9, wherein the carboxylic acid is represented by
Formula (I): R¹-(CH₂)ₙ₁-COOH
(in the formula, R¹ is a methyl group, a carboxyl group, or an aldehyde group, and n¹ is an integer of 1 to 10), or
Formula (II): R²-(CH₂)ₙ₂-COOH
(in the formula, R² is hydrogen, a hydroxyl group, or an amino group, and n² is an integer of 2 to 11).

11. The recombinant polypeptide according to claim 10, wherein in Formula (I), n¹ is an integer of 1 to 7, and
in Formula (II), n² is an integer of 2 to 8.

12. The recombinant polypeptide according to claim 10 or 11, wherein in Formula (I), n¹ is an integer of 2 to 4, and in Formula (II), n² is an integer of 3 to 5.

13. A DNA encoding the recombinant polypeptide according to any one of claims 1 to 12.

14. A genetically modified plasmid comprising the DNA according to claim 13.

15. A recombinant microorganism into which the DNA according to claim 14 is introduced.

16. The recombinant microorganism according to claim 14, wherein the recombinant microorganism is a microorganism having a production ability of at least one selected from the group consisting of dicarboxylic acids having 4 to 12 carbon atoms.

17. A culture of the recombinant microorganism according to claim 15 or 16 and/or an extract of the culture.

18. A method for producing a target compound, the method comprising mixing the recombinant polypeptide according to any one of claims 1 to 12 or the culture and/or the extract of the culture according to claim 17 with a substrate compound to obtain a mixed solution.

19. The method according to claim 18, wherein the substrate compound is represented by
Formula (I): R¹-(CH₂)ₙ₁-COOH
(in the formula, R¹ is a methyl group, a carboxyl group, or an aldehyde group, and n¹ is an integer of 1 to 10), or
Formula (II): R²-(CH₂)ₙ₂-COOH
(in the formula, R² is hydrogen, a hydroxyl group, or an amino group, and n² is an integer of 2 to 11), and
the target compound is an aldehyde.

20. The method according to claim 19, wherein the aldehyde is selected from the group consisting of 4-aminobutan-1-al, 5-aminopentan-1-al, 6-aminohexan-1-al, butanedialdehyde, pentanedialdehyde, hexanedialdehyde, 4-hydroxybutan-1-al, 5-hydroxypentan-1-al, 6-hydroxyhexan-1-al, and glutaraldehyde.

21. The method according to claim 18, wherein the target compound is an alcohol, and
the production method includes adding, to the mixed solution, an enzyme having a catalytic activity of converting an aldehyde into an alcohol to obtain a corresponding alcohol.

22. The method according to claim 18, wherein the target compound is an amine, and
the production method includes adding, to the mixed solution, an enzyme having a catalytic activity of converting an aldehyde into an amine to obtain a corresponding amine.
